(19) Europäisches Patentamt
European Patent Office
Office européen des brevets

(11) Veröffentlichungsnummer: **0 043 917**
**B1**

(12) # EUROPÄISCHE PATENTSCHRIFT

(45) Veröffentlichungstag der Patentschrift: **24.10.84**

(21) Anmeldenummer: **81104529.3**

(22) Anmeldetag: **12.06.81**

(51) Int. Cl.³: **C 07 D 231/18**, C 07 D 405/12, C 07 D 413/12, C 07 D 403/12, A 01 N 47/18, A 01 N 47/24

(54) **N-acylierte N-Methyl-O-pyrazol(4)yl-carbaminsäureester, Verfahren zu ihrer Herstellung und ihre Verwendung in Schädlingsbekämpfungsmitteln.**

(30) Priorität: **25.06.80 DE 3023675**

(43) Veröffentlichungstag der Anmeldung: **20.01.82 Patentblatt 82/3**

(45) Bekanntmachung des Hinweises auf die Patenterteilung: **24.10.84 Patentblatt 84/43**

(84) Benannte Vertragsstaaten: **CH DE FR GB IT LI**

(56) Entgegenhaltungen: **EP - A - 0 023 326**

(73) Patentinhaber: **BAYER AG, Konzernverwaltung RP Patentabteilung, D-5090 Leverkusen 1 Bayerwerk (DE)**

(72) Erfinder: **Mauer, Fritz, Dr., Roeberstrasse 8, D-5600 Wuppertal 1 (DE)**
Erfinder: **Hammann, Ingeborg, Dr., Belfortstrasse 9, D-5000 Koeln (DE)**
Erfinder: **Homeyer, Bernhard, Dr., Obere Strasse 28, D-5090 Leverkusen 3 (DE)**
Erfinder: **Behrenz, Wolfgang, Dr., Untergruendemich 14, D-5063 Overath (DE)**

EP 0 043 917 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue N-acylierte N-Methyl-O-pyrazol(4)yl-carbaminsäureester, Verfahren zu ihrer Herstellung und ihre Verwendung in Schädlingsbekämpfungsmitteln, insbesondere in Insektiziden, Akariziden und Nematiziden.

Es ist bekannt, dass bestimmte N,N-Dimethyl-O-pyrazolyl-carbaminsäureester, wie z.B. N,N-Dimethyl-O-(1-phenyl-3-methyl-pyrazol-5-yl)- und N,N-Dimethyl-O-(1-isopropyl-3-methyl-pyrazol-5-yl)-carbaminsäureester insektizide Eigenschaften aufweisen (vgl. CH-PS 282 655).

Die insektizide Wirkung dieser Verbindungen ist jedoch, insbesondere bei niedrigen Wirkstoffkonzentrationen und Aufwandmengen, nicht immer zufriedenstellend.

Es wurden nun neue N-acylierte N-Methyl-O-pyrazol(4(yl-carbaminsäureester der Formel I gefunden

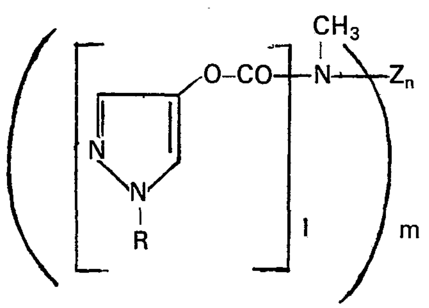

(I)

in welcher

R für gegebenenfalls durch Halogen, Cyano, $C_1$–$C_4$-Alkoxy oder $C_1$–$C_4$-Alkylthio substituiertes $C_1$–$C_5$-Alkyl für $C_3$–$C_5$-Alkenyl, $C_3$–$C_5$-Alkinyl, $C_3$–$C_6$-Cycloalkyl oder für gegebenenfalls halogen-substituiertes Phenyl, Benzyl oder Phenylethyl steht,

Z für die Reste –S–, –S–S–, –CO–$R^1$ oder –$S_r(O)_sR^4$ steht, wobei $R^1$, r, s, $R^4$ die unten angegebene Bedeutung haben und

l für 1 oder 2,

m für 1 oder 2,

n für 0 oder 1 steht,wobei für den Fall, dass 1, m und n jeweils für 1 stehen,

Z für den Rest –CO–$R^1$ steht, worin

$R^1$ für Halogen, $C_1$–$C_4$-Alkoxy, $C_3$–$C_5$-Alkenoxy, $C_3$–$C_5$-Alkinoxy, $C_1$–$C_4$-Alkylthio, $C_1$–$C_4$-Monoalkylamino, $C_1$–$C_4$-Dialkyl-amino, $C_1$–$C_4$-Alkyl-hydroxylamino, für gegebenenfalls durch Halogen, Nitro, Cyano, Trifluormethyl, $C_1$–$C_4$-Alkyl, $C_1$–$C_4$-Alkoxy, $C_1$–$C_4$-Alkylendioxy, $C_1$–$C_4$-Alkylthio, $C_1$–$C_4$-Alkoxy-carbonyl-substituiertes Phenoxy, Phenylthio oder Phenylamino, für 2,3-Dihydro-2,2-dimethyl-7-benzofuranyl oder für den Rest

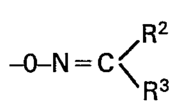

steht, worin

$R^2$ für Wasserstoff, $C_1$–$C_4$-Alkyl oder $C_1$–$C_4$-Dialkylamino-carbonyl steht und

$R^3$ für $C_1$–$C_4$-Alkyl, $C_1$–$C_4$-Alkylthio, Cyano-$C_1$–$C_4$-alkylthio, $C_1$–$C_4$-Alkylthio-$C_1$–$C_4$-alkyl steht oder die beiden Reste $R^2$ und $R^3$ zusammen für gegebenenfalls durch Sauerstoff, Schwefel, SO oder $SO_2$ unterbrochenes $C_2$–$C_8$-Alkandiyl stehen, wobei für den Fall, dass l, m und n jeweils für 1 stehen,

Z für den Rest –$S_r(O)_sR^4$ steht, worin r für 1 oder 2 und s für Null, 1 oder 2 stehen und

$R^4$ für gegebenenfalls durch Halogen substituiertes $C_1$–$C_4$-Alkyı, $C_3$–$C_5$-Alkenyl, $C_3$–$C_5$-Alkinyl oder $C_3$–$C_6$-Cycloalkyl, für gegebenenfalls durch Halogen, Cyano, Nitro, Trifluormethyl, $C_1$–$C_4$-Alkyl oder $C_1$–$C_4$-Alkoxy substituiertes Phenyl, Benzyl oder Phenylethyl oder für den Rest

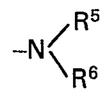

steht, worin

$R^5$ für $C_1$–$C_4$-Alkyl, $C_3$–$C_5$-Alkenyl, $C_3$–$C_5$-Alkinyl, $C_3$–$C_6$-Cycloalkyl oder Benzyl steht und

$R^6$ für $C_1$–$C_4$-Alkyl, $C_3$–$C_5$-Alkenyl, $C_3$–$C_5$-Alkinyl, $C_3$–$C_6$-Cycloalkyl, Benzyl, Phenylethyl, Halogencarbonyl, Formyl, $C_1$–$C_4$-Alkyl-carbonyl, $C_1$–$C_4$-alkoxy-carbonyl, $C_1$–$C_4$-Alkoxy-phenoxy-carbonyl, $C_3$–$C_5$-Alkinoxy-carbonyl, $C_3$–$C_5$-Alkenoxy-carbonyl, $C_1$–$C_4$-Alkylthiocarbonyl, $C_1$–$C_4$-Alkyl-amino-carbonyl, $C_1$–$C_4$-Alkyl-hydroxylaminocarbonyl, $C_1$–$C_{10}$-Alkyl-phenoxy -carbonyl, Di-$C_1$–$C_4$ -alkyl- amino -carbonyl, Phenylthiocarbonyl, Phenoxycarbonyl, 2,3-Dihydro-2,2-dimethyl-7-benzofuranyl-oxycarbonyl, für gegebenenfalls durch Halogen, Cyano, Nitro, Trifluormethyl, $C_1$–$C_{10}$-Alkyl oder $C_1$–$C_4$-Alkoxy substituiertes Phenylsulfenyl, Phenylsulfinyl, Phenylsulfonyl oder Phenyl steht oder für den Rest

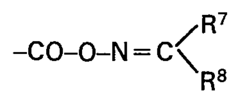

steht, worin

$R^7$ die oben für $R^2$ angegebene und

$R^8$ die oben für $R^3$ angegebene Bedeutung hat,

wobei ferner im Rest 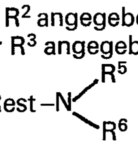 die Reste $R^5$ und $R^6$ zusammen für eine gegebenenfalls durch Sauerstoff oder Schwefel unterbrochene Kohlenwasserstoffkette mit 3 bis 8 Kohlenstoffatomen stehen.

Man erhält die neuen Verbindungen der Formel (I),

(a) wenn man N-Methyl-O-pyrazol(4)yl-carbaminsäureester der Formel II

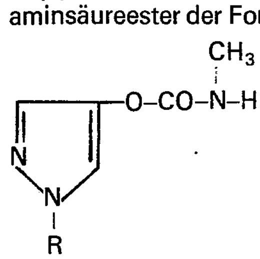

(II)

in welcher

R die oben angegebene Bedeutung hat, mit Acylierungsmitteln der Formel III

$X{-}Z_n$ (III)

in welcher

Z die oben angegebene Bedeutung hat,

X für Fluor, Chlor oder Brom steht und

n für 1 steht, oder, mit Schwefeldichlorid oder Dischwefeldichlorid, gegebenenfalls in Gegenwart eines Säureakzeptors und gegebenenfalls unter Verwendung eines Verdünnungsmittels umsetzt, oder

(b) wenn man 4-Hydroxy-pyrazole der Formel IV

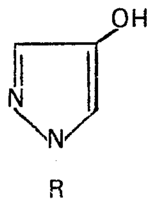

(IV)

in welcher

R die oben angegebene Bedeutung hat, mit Acylierungsmitteln der Formel V

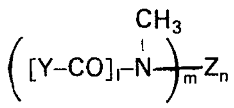

(V)

in welcher

Z, l, m und n die oben angegebene Bedeutung haben und

Y für Fluor oder Chlor steht, gegebenenfalls in Gegenwart eines Säureakzeptors und gegebenenfalls unter Verwendung eines Verdünnungsmittels und gegebenenfalls in Gegenwart einer weiteren carbamoylierbaren Verbindung umsetzt.

Die neuen N-acylierten N-Methyl-0-pyrazol-(4)yl-carbaminsäureester der Formel (I) zeichnen sich durch hohe pestizide Wirksamkeit aus.

Überraschenderweise zeigen die erfindungsgemässen Verbindungen der Formel (I) eine erheblich höhere insektizide, akarizide und nematizide Wirkung als aus dem Stand der Technik bekannte Verbindungen analoger Konstitution und gleicher Wirkungsrichtung.

Die Erfindung betrifft vorzugsweise Verbindungen der Formel (I), in welcher

R die oben angegebene Bedeutung hat und entweder l für 2, m für 1 und n für Null steht, oder, für den Fall, dass Z für Schwefel steht, l für 1, m für 2 und n für 1 steht.

Verwendet man als Ausgangsstoffe bei Verfahren (a) beispielsweise N-Methyl-O-(1-methyl-pyrazol(4)yl)-carbaminsäureester und Trichlormethanolsulfensäurechlorid und bei Verfahren (b) beispielsweise 1-Methyl-4-hydroxy-pyrazol sowie Bis-chlorcarbonylmethylamin, so können die bei den erfindungsgemässen Herstellungsmethoden ablaufenden Reaktionen durch folgende Formelschemata skizziert werden:

(a)

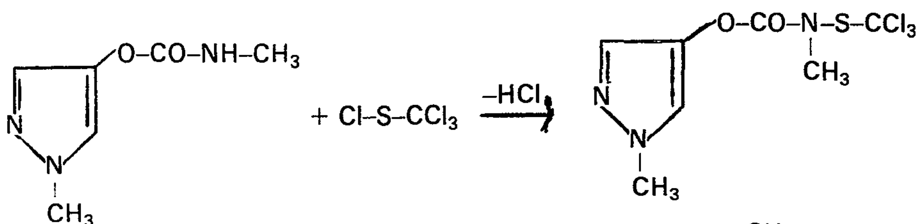


(b)

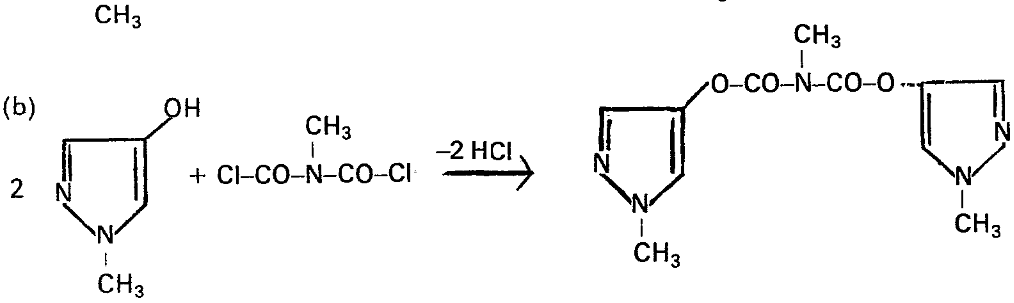


Die bei Verfahren (a) als Ausgangsstoffe zu verwendenden N-Methyl-O-pyrazol(4)yl-carbaminsäureester sind durch Formel (II) definiert.

Als Beispiele für die Verbindungen der Formel (II) seien genannt:

1-Methyl-, 1-Ethyl-, 1-n-Propyl-, 1-iso-Propyl-, 1-(1,1-Dimethylpropyl)-, 1-(2,2-Dimethylpropyl)-, 1-n-Butyl-, 1-iso-Butyl-, 1-sek.-Butyl-, 1-tert. Butyl-, 1-(1-Methylbutyl)-, 1-(2-Methylbutyl)-, 1-(3-Methylbutyl)-, 1-n-Pentyl-, 1-(1-Ethyl-propyl)-, 1-Cyclopropyl-, 1-Cyclobutyl-, 1-Cyclopentyl-, 1-Cyclohexyl, 1-Allyl-, 3-Methyl-2-butenyl-, 1-Propargyl-, 1-Methylthio-methyl, 1-(2-Cyano-ethyl)-, 1-(2-Methoxy-ethyl)-, 1-(2-Ethoxy-ethyl)-, 1-(2-Methylthio-ethyl)-, 1-Chlormethyl-, 1-Dichlormethyl-1-Trichlormethyl-, 1-(2-Chlorethyl)-, 1-Trifluormethyl-, 1-Phenyl-, 1-Benzyl- und 1-Phenetyl-pyrazol(4)yl-N-methyl-carbaminsäureester.

Die Verbindungen der Formel (II) sind Gegenstand einer nicht vorveröffentlichten Patentanmeldung (vgl. DE-OS 2 931 033).

Man erhält die Verbindungen der Formel (II) durch Umsetzung von 4-Hydroxy-pyrazolen der Formel (IV) mit Methylisocyanat in Gegenwart eines Verdünnungsmittels, wie z.B. Aceton und

gegebenenfalls in Gegenwart eines Katalysators, wie z.B. Triethylamin, bei Temperaturen zwischen 10 und 80 °C.

Die bei Verfahren (a) weiter als Ausgangsstoffe zu verwendenden Acylierungsmittel sind durch Formel (III) definiert. Vorzugsweise hat darin

Z die gleiche Bedeutung, wie sie oben bei der Definition des Restes Z in Formel (I) angegeben, weiter stehen vorzugsweise

X für Fluor oder Chlor, und

n für 1.

Als Beispiele für die Verbindungen der Formel (III) seien genannt:

Schwefeldichlorid, Dischwefeldichlorid, Methansulfensäurechlorid, Trichlormethansulfensäurechlorid, Dimethylamino-, Di-n-butyl-aminosulfensäurechlorid und Morpholinosulfensäurechlorid.

Verbindungen der Formel (III) sind bekannt (vgl. DE-OS 2 433 680).

Die bei Verfahren (b) als Ausgangsstoffe zu verwendenden 4-Hydroxypyrazole sind durch Formel (IV) definiert.

Als Beispiele für die Verbindungen der Formel (IV) seien genannt:

1-Methyl-, 1-Ethyl-, 1-n-propyl-, 1-iso-Propyl-, 1-(1,1-Dimethylpropyl)-, 1-(2,2-Dimethylpropyl-, 1-n-Butyl-, 1-iso-Butyl-, 1-sek.-Butyl-, 1-tert.-Butyl-, 1-(1-Methylbutyl), 1-(2-Methylbutyl)-, 1-(3-Methylbutyl)-, 1-n-Pentyl-, 1-(1-Ethyl-propyl)-, 1-Cyclopropyl-, 1-Cyclobutyl-, 1-Cyclopentyl-, 1-Cyclohexyl-, 1-Allyl-, 3-Methyl-2-butenyl-, 1-Propargyl-, 1-Methylthio-methyl-, 1-(2-Cyano-ethyl)-1-2-Methoxy-ethyl)-, 1-(2-Ethoxy-ethyl)-, 1-(2-Methylthio-ethyl)-, 1-Chlormethyl-, 1-Dichlormethyl-, 1-Trichlormethyl-, 1-(2-Chlorethyl)-, 1-Trifluor-methyl-, 1-Phenyl-, 1-Benzyl- und 1-Phenethyl-4-hydroxy-pyrazol.

Die 4-Hydroxypyrazole der Formel (IV) sind teilweise bekannt (vgl. Liebigs Ann. Chem. 313 (1900), 17), und teilweise Gegenstand einer nicht vorveröffentlichten Patentanmeldung (DEOS 2 931 033).

Man erhält die 4-Hydroxy-pyrazole beispielsweise durch Umsetzung der entsprechenden Methoxypyrazole mit Bromwasserstoffsäure. Die Herstellung der 4-Methoxy-pyrazole kann auf bekannte Weise, beispielsweise durch Umsetzung von entsprechenden Hydrazinen mit 2-Methoxy-4-dimethyl-amino-acrolein erfolgen (vgl. Archiv der Pharmazie 300, (1967), 704–708).

Die bei Verfahren (b) weiter als Ausgangsstoffe zu verwendenden Acylierungsmittel sind durch Formel (V) definiert. Vorzugsweise hat darin

Z die gleiche Bedeutung, wie sie oben bei der Definition des Restes Z in Formel (I) angegeben ist, weiter stehen vorzugsweise

Y für Fluor oder Chlor

l für 1 oder 2,

m für 1 oder 2 und

n für Null oder 1.

Als Beispiele für die Verbindungen der Formel (V) seien genannt:

Bis-chlorcarbonyl-methylamin, Bis-(N-methyl-N-fluorcarbonylamino)- sulfid, N-Methyl-N-(1-methylthioacetaldehyd-O-(methylcarbamoylsulfenyl)-oxim)-carbamoylfluorid, N-Methyl-N-trichlormethylsulfenyl-carbamoylfluorid, N-Methyl- N-chloridfluormethylsulfenyl -carbamoylfluorid, N-Methyl -N- fluordichlormethylsulfenyl-carbamoylfluorid, N-Methyl -N-phenylsulfenyl-carbamoylfluorid, N,N'-Bis-fluorcarbonyldithio-bis-methylamin, N-Methyl -N-methylthiosulfenyl -carbamoylfluorid, N-Methyl-N-(2-methyl-2-propanthio-sulfenyl)-carbamoylfluorid, N-Methyl-N-morpholinosulfenyl-carbamoylfluorid, N-Methyl-N-(N-methyl-N-formyl-amino-sulfenyl)-carbamoylfluorid, N-Methyl-N- (N-methyl-N-acetyl-aminosufenyl) -carbamoylfluorid und (N- Methyl -N- (N'-fluorcarbonyl -N'- methylaminosulfenyl)- carbamoyloxy -4- nonylbenzol.

Verbindungen der Formel (V) sind bekannt (vgl. DE-AS 1 297 095, DE-OS 2 425 211 und 2 530 278; DE-OS 1 932 830, 2 530 278, 2 635 883, 2 654 282 und 2 828 133; US-PS 4 181 734).

Carbamoylierbare Verbindungen, welche gegebenenfalls bei Verfahren (b) eingesetzt werden, sind vorzugsweise

(a) Phenole, Thiophenole, Phenylamine, Hydroxy-pyrazole und Hydroxy-pyrimidine, welche jeweils gegebenenfalls substituiert sind durch Halogen, Cyano, Nitro, $C_1$–$C_4$-Alkylcarbonyl, $C_1$–$C_4$-Alkoxy-carbonyl, $C_1$–$C_4$-Alkyl-amino-carbonyl, $C_1$–$C_4$-Alkyl-amino und/oder durch gegebenenfalls halogen-substituierte Reste aus den Reihe

$C_1$–$C_5$-Alkyl, $C_3$–$C_6$-Cycloalkyl, $C_1$–$C_4$-Alkoxy, $C_1$–$C_4$-Alkylthio, $C_1$–$C_4$-Alkylsulfinyl, $C_1$–$C_4$-Alkyl-sulfonyl, $C_1$–$C_4$-Alkoxy-$C_1$–$C_2$-alkyl, $C_1$–$C_4$-Alkylthio-$C_1$–$C_2$-alkyl, $C_1$–$C_4$ -Alkylsulfinyl-$C_1$–$C_2$-alkyl, $C_1$–$C_4$ -Alkylsulfonyl- $C_1$–$C_2$-alkyl, $C_2$–$C_4$ -Alkandiyl, $C_2$–$C_4$-Oxaalkandiyl und/oder $C_1$–$C_4$-Dioxaalkandiyl,

(b) Oxime von Aldehyden mit bis zu 8 Kohlenstoffatomen, welche gegebenenfalls durch Halogen, Cyano, Dimethylcarbamoyl, $C_1$–$C_4$-Alkylthio und/oder $C_1$–$C_4$-Alkylsulfonyl substituiert sind, oder

(c) aliphatische, gesättigte oder ungesättigte Alkohole und Amine mit bis zu 6 Kohlenstoffatomen.

Als Beispiele für die carbomoylierbaren Verbindungen seien genannt:

Thiophenol, 1-Methylthio-acetaldehydoxim, Propargylalkohol, Phenol, Dimethylamin, Ethanol, Allylalkohol, 3,3-Dimethyl-allylalkohol, 2,3-Dihydro -2,2- dimethyl-7- hydroxy- benzofuran, 2- Methylthio-isobutyraldehydoxim, 2-Isopropoxy-phenol, Ethylmercaptan, Isopropylalkohol, O,N- Dimethyl-hydroxylamin, Isopropylamin, 3- Methyl -2- buten -1-01, Morpholin, 2-Methyl -2- methylthio-propanaldoxim, 1- Methyl -thio-1- dimethylamino- carbonyl- methanaldoxim, 3,3- Dimethyl -1- methylthio-butan-2-on-oxim und 2,3- (Dimethylmethyldioxy)-phenol sowie die als Beispiele für 4-Hydroxypyrazole der Formel (IV) oben angeführten Verbindungen.

Die erfindungsgemässen Herstellungsverfahren (a) und (b) werden vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methylisopropyl- und Methyl-isobutylketon, Ester, wie Essigsäuremethylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Vorzugsweise wird Toluol als Verdünnungsmittel verwendet.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin und Diazabicycloundecen.

Triethylamin wird bevorzugt als Säureakzeptor eingesetzt.

Die Reaktionstemperatur kann innerhalb eines grösseren Bereichs variiert werden. Im allgemeinen arbeitet man zwischen −20 und 100 °C, vorzugsweise bei 0 bis 80 °C.

Die erfindungsgemässen Verfahren werden im allgemeinen bei Normaldruck durchgeführt.

Zur Durchführung der erfindungsgemässen Verfahren werden die Ausgangsstoffe gewöhnlich in äquivalenten Mengen eingesetzt. Ein Überschuss der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Danach gibt man gegebenenfalls ein mit Wasser nicht mischbares organischen Lösungsmittel, z.B. Toluol, zu und arbeitet die organische Phase wie üblich durch Waschen, Trocknen und Abdestillieren des Lösungsmittels auf.

Die neuen Verbindungen fallen zum Teil in Form von Ölen an, die sich zum Teil nicht unzersetzt destillieren lassen, jedoch durch sogenanntes «Andestillieren», d.h. durch längeres Erhitzen unter vermindertem Druck auf mässig erhöhte Temperaturen von den letzten flüchtigen Anteilen befreit und auf diese Weise gereinigt werden. Zu ihrer Charakterisierung dient der Brechungsindex.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpohagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoas ca spp., Euscelis bilobatus, Nephotettix cincticeps, Lecanicum corni, Saisettia oleae, Laodelphax striatellus, Nilaparvata lugens, Aonidiella auranti, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotacsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Antrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocama spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora Boophilus spp., Rhipicephalus spp., Amblyomma spp., Hyallomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphtaline, chlorierte Aromaten oder chlorierte aliphatische Kohlenwasserstoffe, wie Chlorbenzole, Chlorätyhlene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methylläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkyl- sulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie angeordnete Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffe, Sterilantien, Akariziden, Nematiziden, Fungiziden, wachstumsregulierenden Stoffen oder Herbiziden vorliegen. Zu den Insektiziden zählen beispielsweise

Phosphorsäureester, Carbamate, Carbonsäureester, chlorierte Kohlenwasserstoffe oder Phenylharnstoffe,

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Ekto- und Endoparasiten auf dem veterinärmedizinischen Gebiet.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuders sowie durch parenterale Anwendung in Form beispielsweise der Injektion.

Herstellungsbeispiele

Beispiel 1

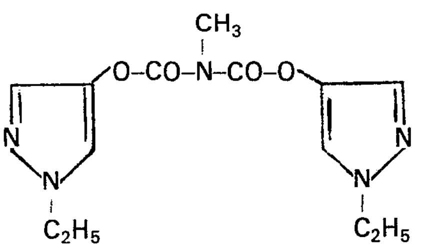

(1)

Zu einer Mischung aus 5,6 g (0,05 Mol) 1-Ethyl-4-hydroxypyrazol, 3,9 g (0,025 Mol) Bis-Chlorcarbonylmethylamin und 100 ml Toluol tropft man 5,1 g (0,05 Mol) Triethylamin. Nach 10 Stunden wird zweimal mit 25 ml Wasser gewaschen. Die organische Phase trocknet man über Natriumsulfat und destilliert dann das Lösungsmittel im Vakuum ab. Der Rückstand wird im Hochvakuum andestilliert. Man erhält auf diese Weise 7 g (91% g der Theorie) Bis-[1-ethylpyrazol(4)yl-oxy]-carbonyl-methylamin in Form eines hellbraunen Öles mit dem Brechungsindex $n_D^{20}$ : 1,5009

Beispiel 2

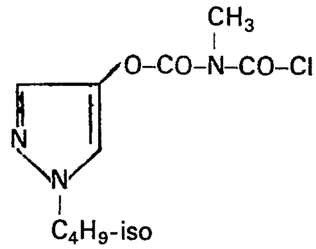

(2)

Eine Lösung von 7 g (0,05 Mol) 1-iso-Butyl-4-hydroxypyrazol und 7,8 g (0,05 Mol) Bis-Chlorcarbonyl-methylamin in 100 ml Toluol wird mit 5,1 g (0,05 Mol) Triethylamin versetzt und 8 Stunden bei Raumtemperatur gerührt. Dann filtriert man vom ausgefallenen Aminsalz, wäscht mitToluol nach und dampft das Filtrat ein. Zurück bleiben 10 g (77% der Theorie) N-Chlorcarbonyl-N-[iso-butyl-pyrazol(4)yl-oxy] -carbonyl-methylamin in Form eines hellbraunen Öles mit dem Brechungsindex Nn $_D^{24}$ :1,5043.

Beispiel 3

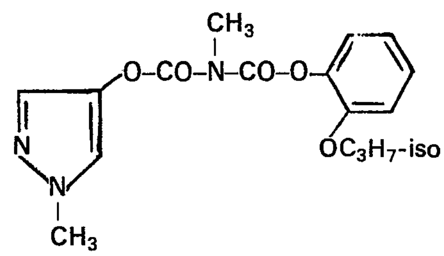

(3)

Zu einer Lösung von 4 g (0,04 Mol) 1-Methyl-4-hydroxypyrazol und 6,25 g (0,04 Mol) Bis-Chlorcarbonylmethylamin in 100 ml Toluol tropft man 4,1 g (0,04 Mol) Triethylamin und rührt 4 Stunden bei Raumtemperatur nach. Dann gibt man 6,1 g (0,04 Mol) 2-Isopropoxyphenol und weitere 4,1 g (0,04 Mol) Triethylamin zu und rührt wieder 4 Stunden nach. Dann wäscht man das Reaktionsgemisch zweimal mit je 25 ml Wasser, trocknet die organische Phase über Natriumsulfat und destilliert das Lösungsmittel im Vakuum ab. Der Rückstand wird im Hochvakuum andestilliert. Man erhält so 12,5 g (94% der Theorie) N-[(2-Isopropoxy)-phenoxy]carbonyl-N-[1-methyl-pyrazol (4)yl-oxy]- carbonyl-methylamin in Form eines gelben Öles mit dem Brechungsindex $n_D^{24}$ :1,5271.

Beispiel 4

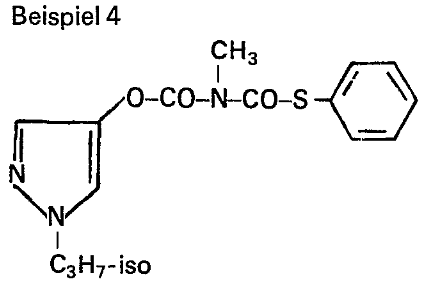

(4)

Zu einer Lösung von 9,8 g (0,04 Mol) N-Chlorcarbonyl-N- [1-iso-propyl-pyrazol (4)yl-

oxy]-carbonylmethylamin – welches man analog Beispiel 2 dargestellt hat – und 4,4 g (0,04 Mol) Thiophenol in 100 ml Toluol tropft man 4 g (0,04 Mol) Triethylamin. Man rührt das Gemisch 4 Stunden nach Raumtemperatur nach und schüttelt es dann zweimal mit je 25 ml Wasser aus. Die organische Phase wird über Natriumsulfat getrocknet und im Vakuum eingedampft. Den Rückstand destilliert man im Hochvakuum an. Man erhält so 12 g (94% der Theorie) N-Phenylthiocarbonyl-N- [1-iso-propyl-pyrazol (4)yl-oxy)]-carbonyl-methylamin als braunes Öl mit dem Brechungsindex $n_D^{20}$ :1,5662.

Analog einem der Beispiele 1 bis 4 werden die folgenden Verbindungen der Formel

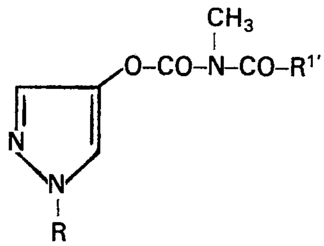

(Ia)

hergestellt:

| Produkt Nr. | R | R1' | Ausbeute (% der Theorie) | Physikal. Daten (Brechungsindex; Schmelzpunkt °C) |
|---|---|---|---|---|
| 5 | $CH_3$ | 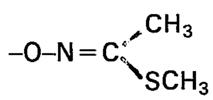 | | |
| 6 | $C_3H_7$-iso | 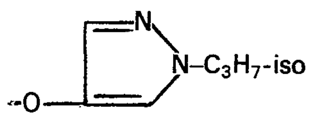 | 84 | $n_D^{20}$:1,5089 |
| 7 | $C_4H_9$-n | 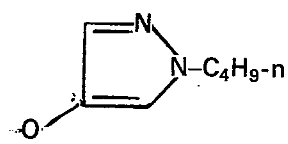 | 77 | $n_D^{20}$:1,5055 |
| 8 | $C_3H_7$-iso | Cl | 98 | $n_D^{20}$: 1,5077 |
| 9 | $C_3H_7$-iso | $-O-CH_2-C=CH$ | 91 | $n_D^{23}$:1,5004 |
| 10 | $C_4H_9$-iso | 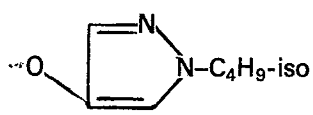 | 77 | $n_D^{20}$:1,5078 |
| 11 | $C_3H_7$-iso | 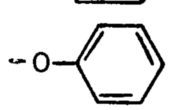 | 91 | $n_D^{20}$:1,5325 |
| 12 | $CH_2=CH-CH_2$ | $N(CH_3)$ | | |
| 13 | $C_4H_9$-tert. | $-N(CH_3)_2$ | | |
| 14 | $C_3H_7$-iso | $-OC_2H_5$ | 98 | $n_D^{20}$:1,4680 |
| 15 | $CH=C-CH_2$ | $-O-CH_2-CH=CH_2$ | | |
| 16 | $CH_3-S-CH_2$ | 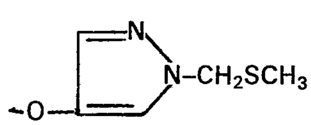 | | |
| 17 | $C_3H_7$-iso | $-N(CH_3)_2$ | 87 | $n_D^{20}$:1,5011 |

(Fortsetzung)

| Produkt Nr. | R | $R^{1'}$ | Ausbeute (% der Theorie) | Physikal. Daten (Brechungsindex; Schmelzpunkt °C) |
|---|---|---|---|---|
| 18 | $C_4H_9$-sek. | 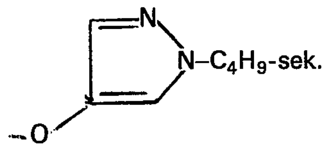 | 77 | $n_D^{20}$:1,5001 |
| 19 | $C_4H_9$-tert. | $-O-CH_2-C=CH$ | | |
| 20 | $C_3H_7$-iso | 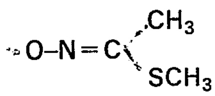 | | $n_D^{20}$:1,5047 |
| 21 | $C_2H_5$ | 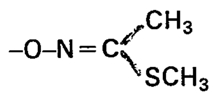 | | |
| 22 | $C_3H_7$-iso | $-O-CH_2-CH=CH_2$ | 84 | $n_D^{20}$:1,4953 |
| 23 | 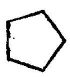 | 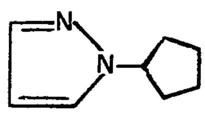 | | |
| 24 | $CH_3$ | 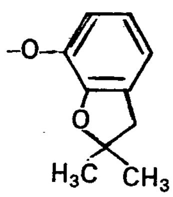 | | |
| 25 | $C_3H_7$-iso | 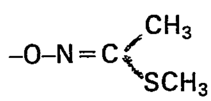 | 80 | $n_D^{23}$:1,5304 |
| 26 | $C_2H_5O-CH_2-CH_2$ | 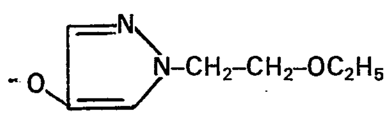 | | |
| 27 | $C_4H_9$-sek. | 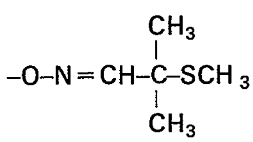 | | |
| 28 | $CH(C_2H_5)_2$ | 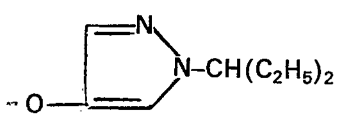 | | |
| 29 | $C_3H_7$-iso | 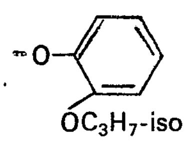 | 83 | $n_D^{23}$:1,5181 |
| 30 | 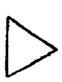 | 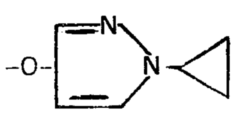 | | |

(Fortsetzung)

| Produkt Nr. | R | $R^{1'}$ | Ausbeute (% der Theorie) | Physikal. Daten (Brechungsindex; Schmelzpunkt °C) |
|---|---|---|---|---|
| 31 | $C_3H_7$-iso | $-SC_2H_5$ | 83 | $n_D^{23}$: 1,5215 |
| 32 | $C_4H_9$-tert. | 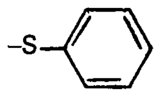 | | |
| 33 | $C_3H_7$-iso | $-OC_3H_7$-iso | 93 | $n_D^{23}$:1,4930 |
| 34 | $C_2H_5$ | 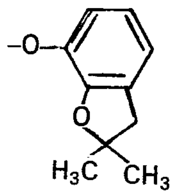 | | |
| 35 | $CH_3-S-CH_2$ | 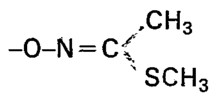 | | |
| 36 | $C_3H_7$-iso | 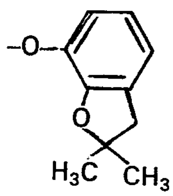 | 87 | $n_D^{23}$:1,5312 |
| 37 | $C_4H_9$-tert. | 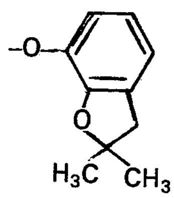 | 86 | $n_D^{24}$: 1,5264 |
| 38 | $C_3H_7$-n | 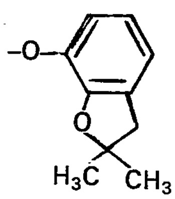 | | |
| 39 | $C_3H_7$-iso | 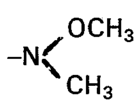 | 83 | $n_D^{20}$:1,4958 |
| 40 | $C_4H_9$-tert. | 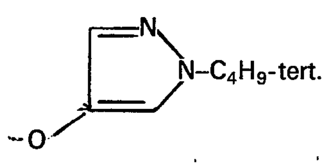 | 77 | $n_D^{23}$:1,5089 |
| 41 | $CH_3$ | $-CH_2-CH=CH_2$ | | |
| 42 | $C_2H_5$ | $-NH-C_3H_7$-iso | | |
| 43 | $C_4H_9$-sek. | $-NH-C_3H_7$-iso | | |
| 44 | $C_4H_9$-tert. | Cl | 88 | $n_D^{22}$:1,5057 |
| 45 | $C_4H_9$-tert. | $-O-CH_2-CH=C(CH_3)_2$ | | |

(Fortsetzung)

| Produkt Nr. | R | $R^{1'}$ | Ausbeute (% der Theorie) | Physikal. Daten (Brechungsindex; Schmelzpunkt °C) |
|---|---|---|---|---|
| 46 | $C_4H_9$-sek. | 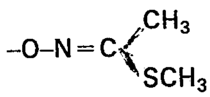 | | |
| 47 | $C_4H_9$-tert. | 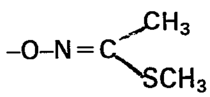 | | |
| 48 | $C_3H_7$-iso | $-O-CH_2-CH=C(CH_3)_2$ | 81 | $n_D^{25}$:1,49 49 |
| 49 | $C_3H_7$-iso | $-NH-C_3H_7$-iso | 97 | $n_D^{23}$:1,4906 |
| 49a | $C_3H_7$-iso | $CH_3$ | 88 | $n_D^{18}$:1,4957 |
| 49b | $C_4H_9$-sec. | $CH_3$ | 84 | $n_D^{25}$:1,4889 |

Beispiel 5

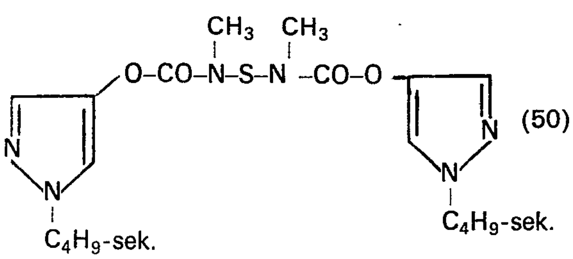

(50)

Zu einer Mischung aus 8,4 g (0,06 Mol) 1-sek. Butyl-4-hydroxy-pyrazol, 80 ml Toluol und 5,5 g (0,03 Mol) Bis-N-methyl-N-fluorcarbonylamino)-sulfid tropft man bei 20-25 °C 6,2 g (0,06 Mol) Triethylamin und rührt anschliessend 10 Stunden bei Raumtemperatur nach. Dann schüttelt man zweimal mit je 25 ml Wasser aus, trocknet die organische Phase über Natriumsulfat und destilliert das Lösungsmittel im Vakuum ab. Zurück bleiben 10 g (79% der Theorie) Bis-[N-methyl -N- (1-sek.-butyl-pyrazol(4)yl-oxy-]carbonylamino]- sulfid inForm eines gelben Öles mit dem Brechungsindex $n_D^{19}$ :1,5233.

Beispiel 6

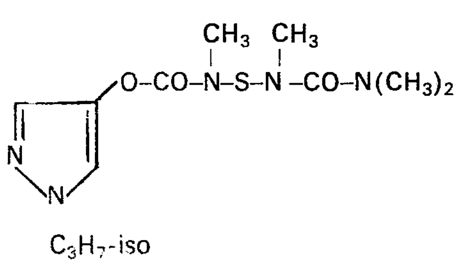

(51)

Eine Lösung von 9,2 g (0,05 Mol) Bis-(N-methyl-N-fluor-carbonylamino)-sulfid in 50 ml Toluol wird bei 0-5 °C mit einer Mischung aus 6,3 g (0,05 Mol) 1-Isopropyl-4-hydroxypyrazol, 5,1g (0,05 Mol) Triethylamin und 200 ml Toluol versetzt. Das Reaktionsgemisch wird 2 Stunden bei Raumtemperatur nachgerührt und dann bei 15-20 °C mit 11 g einer ca. 45%igen wässrigen Dimethylaminlösung versetzt. Man rührt 3 Stunden bei Raumtemperatur nach, trennt die wässrige Phase ab und schüttelt die Toluollösung noch zweimal mit je 25 ml Wasser aus. Die organische Phase wird über Natriumsulfat getrocknet und im Vakuum eingedampft. Der Rückstand wird im Hochvakuum andestilliert. Man erhält so 13,5 g (86% der Theorie) 0-[1-Isopropyl-pyrazol-(4)yl]-N-methyl -N- (N-dimethylamino-carbonyl-N- methyl- aminosulfenyl)-carbaminsäureester in Form eines hellbraunen Öles mit dem Brechungsindex $n_D^{22}$ : 1,5192.

Beispiel 7

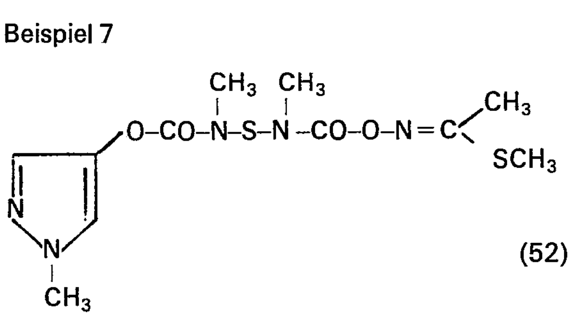

(52)

Zu einer Mischung aus 6,7 g (0,025 Mol) N-Methyl-N- [1-methylthio-acetaldehyd-O-(methylcarbamoylsulfenyl) -oxim] -carbamoylfluorid

(Darst. s. DOS 2 824 394), 2,5 g (0,025 Mol) 1-Methyl-4-hydroxypyrazol und 50 ml Toluol tropft man eine Lösung von 2,5 g (0,025 Mol) Triethylamin in 10 ml Toluol. Man rührt 24 Stunden bei Raumtemperatur nach, schüttelt zwei mal mit je 25 ml Wasser aus und trocknet die organische Phase über Natriumsulfat. Dann destilliert man das Lösungsmittel im Vakuum ab. Man erhält auf diese Weise 6 g (69% der Theorie) O-[1-Methyl-pyrazol(4)yl] -N- methyl -N[1-methylthio-acetaldehyd -O- (methylcarbamoyl -sulfenyl)-oxim]-carbaminsäureester in Form gelber Kristalle mit dem Schmelzpunkt 87 °C.

Analog einem der Beispiele 5-7 können die folgenden Verbindungen der Formel

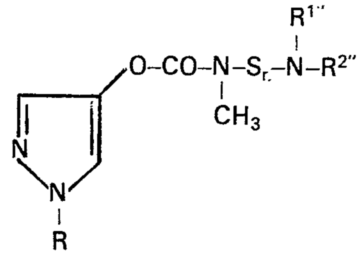

(Ib)

hergestellt werden:

| Produkt Nr. | R | R1'' | R2'' | r | Ausbeute (% der Theorie) | Physikal. Daten (Brechungsindex; Schmelzpunkt °C) |
|---|---|---|---|---|---|---|
| 53 | $C_4H_9$-tert. | | $-CH_2-CH_2-O-CH_2-CH_2-$ | 1 | 57 | $n_D^{26}$:1,5218 |
| 54 | $C_2H_5$ | $CH_3$ | $-CO-N(CH_3)_2$ | 1 | | |
| 55 | $C_3H_7$-iso | $CH_3$ | $-CO-OCH_3$ | 1 | 66 | $n_D^{18}$:1,5427 |
| 56 | $CH_3$ | $CH_3$ | 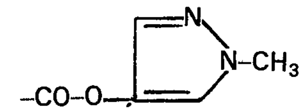 | 1 | | |
| 57 | $C_3H_7$-iso | $CH_3$ | 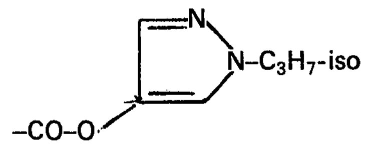 | 1 | 70 | 65–67 |
| 58 | $CH_2=CH-CH_2$ | $CH_3$ | 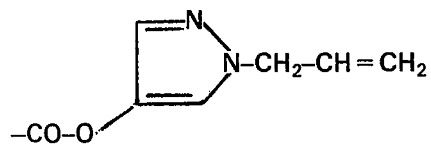 | 1 | | |
| 59 | $C_3H_7$-iso | $C_4H_9$-n | $-C_4H_9$-n | 1 | | |
| 60 | $C_4H_9$-sek. | $CH_3$ | $-CH_3$ | 1 | | |
| 61 | $C_3H_7$-iso | $CH_3$ | $-CO-O-CH_2-CH=CH_2$ | 1 | 84 | $n_D^{22}$: 1,5098 |
| 62 | $C_2H_5$ | $CH_3$ | 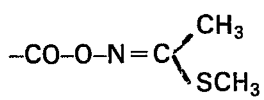 | 1 | | |
| 63 | $C_3H_7$-iso | $CH_3$ | 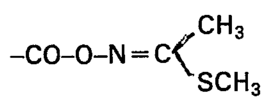 | 1 | 96 | $n_D^{20}$:1,5405 |
| 64 | 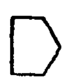 | $CH_3$ | 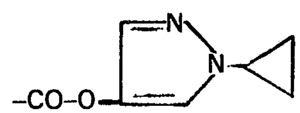 | 1 | | |
| 65 | $CH_3-S-CH_2$ | $CH_3$ | 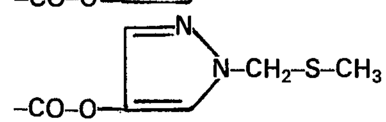 | 1 | | |

(Fortsetzung)

| Produkt Nr. | R | R1″ | R2″ | r | Ausbeute (% der Theorie) | Physikal. Daten (Brechungsindex; Schmelzpunkt °C) |
|---|---|---|---|---|---|---|
| 66 | $C_3H_7$-iso | $CH_3$ | $-CO-OC_3H_7$-iso | 1 | | |
| 67 | $C_4H_9$-tert. | $CH_3$ | 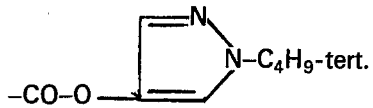 | 1 | 63 | 60–62 |
| 68 | 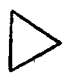 | $CH_3$ | 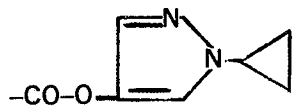 | 1 | | |
| 69 | $C_3H_7n$ | $CH_3$ | 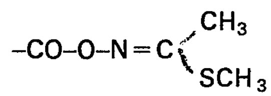 | 1 | | |
| 70 | $C_3H_7$-iso | $CH_3$ | $-CO-SC_2H_5$ | 1 | 83 | $n_D^{22}$:1,5288 |
| 71 | $C_3H_7$-iso | $-CH_2-CH_2-O-CH_2-CH_2-$ | | 1 | 60 | $n_D^{26}$:1,5223 |
| 72 | $CH=C-CH_2$ | $CH_3$ | 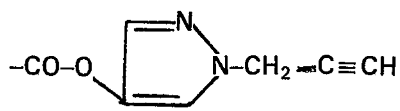 | 1 | | |
| 73 | $C_4H_9$-sek. | $CH_3$ | $-CO-N(CH_3)_2$ | 1 | | |
| 74 | $CH_3-S-CH_2$ | $-C_4H_9$-n | $C_4H_9$-n | 1 | | |
| 75 | $C_3H_7$-iso | $CH_3$ | 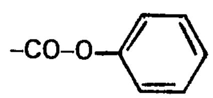 | 1 | 93 | $n_D^{22}$:1,5320 |
| 76 | $C(CH_3)_2-CH_2-CH_3$ | $CH_3$ | 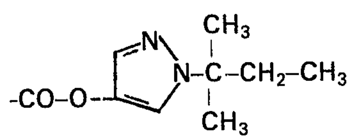 | 1 | 97 | $n_D^{23}$:1,5112 |

(Fortsetzung)

| Produkt Nr. | R | $R^{1''}$ | $R^{2''}$ | r | Ausbeute (% der Theorie) | Physikal. Daten (Brechungsindex; Schmelzpunkt °C) |
|---|---|---|---|---|---|---|
| 77 | $C_4H_9$-tert. | $CH_3$ | $-CO-F$ | 1 | 95 | $n_D^{24}$:1,5037 |
| 78 | $NC-CH_2-CH_2$ | $CH_3$ | 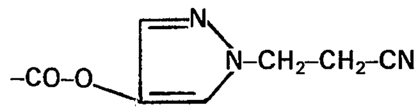 | 1 | | |
| 79 | $C_3H_7$-iso | $CH_3$ | 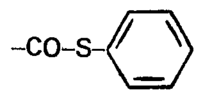 | 1 | 85 | $n_D^{22}$:1,5767 |
| 80 | $C_4H_9$-n | $CH_3$ | 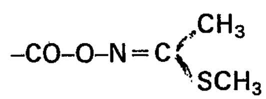 | 1 | | |
| 81 | $C_4H_9$-tert. | $CH_3$ | $-CO-O-CH_2-C{\equiv}CH$ | 1 | 88 | $n_D^{23}$:1,5121 |
| 82 | $C_2H_5$ | $CH_3$ | 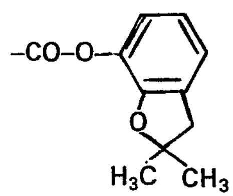 | 1 | | |
| 83 | $C_3H_7$-iso | $CH_3$ | 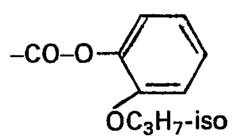 | 1 | 89 | $n_D^{22}$:1,5198 |
| 84 | $C_2H_5O-CH_2-CH_2$ | $CH_3$ | 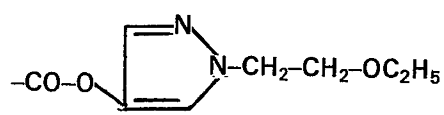 | 1 | | |

(Fortsetzung)

| Produkt Nr. | R | $R^{1''}$ | $R^{2''}$ | r | Ausbeute (% der Theorie) | Physikal. Daten (Brechungsindex; Schmelzpunkt °C) |
|---|---|---|---|---|---|---|
| 85 | $C_3H_7$-iso | $CH_3$ | $-CO-O-$ 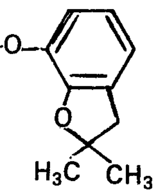 | 1 | 98 | $n_D^{22}$:1,5111 |
| 86 | $C_4H_9$-sek. | $C_4H_9$-n | $-C_4H_9$-n | 1 | | |
| 87 | $C_4H_9$-iso | $C_4H_9$-n | $-C_4H_9$-n | 1 | | |
| 88 | $C_3H_7$-iso | $CH_3$ | $-CO-O-N=C{<}^{CH_3}_{CH_3}$ | 1 | 87 | $n_D^{23}$:1,5179 |
| 89 | $C_4H_9$-tert. | $CH_3$ | $-CO-N(CH_3)_2$ | 1 | | |
| 90 | $C_3H_7$-iso | $CH_3$ | $-CO-N{<}^{CH_3}_{OCH_3}$ | 1 | 83 | $n_D^{23}$:1,5123 |
| 91 | $CH(C_2H_5)_2$ | $CH_3$ | $-CO-O-N=C{<}^{CH_3}_{SCH_3}$ | 1 | | |
| 92 | $C_3H_7$-iso | $CH_3$ | $-CO-F$ | 1 | 95 | $n_D^{24}$:1,5059 |
| 93 | $C_4H_9$-tert. | $CH_3$ | $-CO-O-N=C{<}^{CH_3}_{SCH_3}$ | 1 | 92 | $n_D^{20}$:1,5350 |
| 94 | $C_3H_7$-iso | $CH_3$ | $-CO-O-N=CH-\overset{CH_3}{\underset{CH_3}{C}}SCH_3$ | 1 | | |

(Fortsetzung)

| Pro-dukt Nr. | R | R1'' | R2'' | r | Ausbeute (% der Theorie) | Physikal. Daten (Brechungsindex; Schmelzpunkt °C) |
|---|---|---|---|---|---|---|
| 95 | 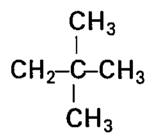 | $CH_3$ | –COO– 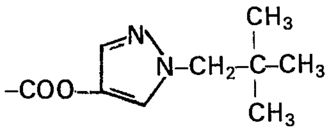 | 1 | | |
| 96 | $C_3H_7$-iso | $CH_3$ | –CO–O– 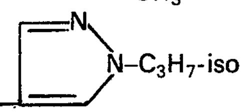 | 2 | 94 | $n_D^{19}$:1,5389 |
| 97 | $C_4H_9$-tert. | $CH_3$ | –CO–O– 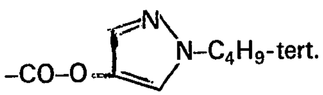 | 2 | 88 | $n_D^{19}$:1,5308 |
| 98 | $C_3H_7$-iso | $CH_3$ | –CO–F | 2 | 93 | $n_D^{19}$:1,5297 |
| 99 | $CH_2$–$CH_2$-Br | $CH_3$ | –CO–F | 1 | 85 | $n_D^{22}$:1,5320 |
| 100 | 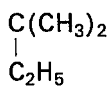 | $CH_3$ | –CO–O– 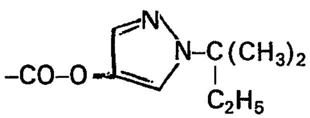 | 2 | 91 | $n_D^{22}$:1,5250 |
| 101 | $C_3H_7$-iso | $CH_3$ | –CO–O–$CH_2$–C≡CH | 1 | 84 | $^{23}_{D}$:1,5139 |
| 102 | $C_4H_9$-sek. | $CH_3$ | –CO–NH–$C_3H_7$-iso | 1 | | |
| 103 | $C_3H_7$-iso | $CH_3$ | –CO–NH–$C_3H_7$-iso | 1 | 84 | $n_D^{23}$:1,5111 |
| 104 | $C_4H_9$-tert. | $CH_3$ | –CO–O–$CH_2$–CH=C$(CH_3)_2$ | 1 | | |
| 105 | $C_3H_7$-iso | $CH_3$ | –CO–O–$CH_2$–$CH_2$–CH=C$(CH_3)_2$ | 1 | 84 | $n_D^{25}$:1,5061 |
| 106 | $C_3H_7$-iso | $CH_3$ | –CO–O$C_2H_5$ | 1 | 87 | $n_D^{23}$:1,5048 |
| 107 | $C_4H_9$-tert. | $CH_3$ | –CO–O–$CH_2$–CH=$CH_2$ | 1 | 80 | $n_D^{23}$:1,5071 |
| 108 | $C_3H_7$-iso | $CH_3$ | –CO–O– 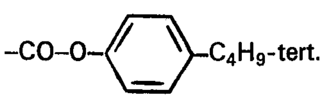 | 1 | 83 | $n_D^{27}$:1,5262 |

(Fortsetzung)

| Pro-dukt Nr. | R | R1'' | R2'' | r | Ausbeute (% der Theorie) | Physikal. Daten (Brechungsindex; Schmelzpunkt °C) |
|---|---|---|---|---|---|---|
| 109 | $C_3H_7$-iso | $CH_3$ | 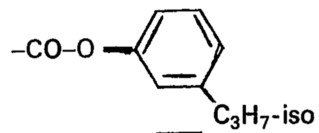 | 1 | 98 | $n_D^{27}$:1,5279 |
| 110 | $C_3H_7$-iso | $CH_3$ | 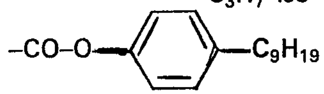 | 1 | 97 | $n_D^{26}$:1,5182 |
| 111 | $C_4H_9$-tert | $CH_3$ | 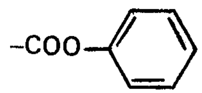 | 1 | 93 | $n_D^{20}$:1,5320 |
| 112 | $C_4H_9$-tert | $CH_3$ | $-CO-NH-C_3H_7$-iso | 1 | 80 | $n_D^{20}$:1,5119 |
| 113 | $C_4H_9$-tert | $CH_3$ | 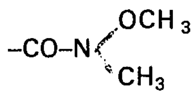 | 1 | 87 | $n_D^{20}$:1,5123 |
| 114 | $CH_2CH_2Br$ | $CH_3$ | 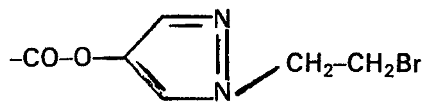 | 1 | 76 | $n_D^{23}$:1,5336 |
| 115 | $C_3H_7$-iso | $CH_3$ | 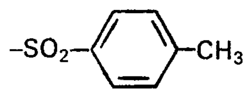 | 1 | 88 | $n_D^{22}$ :1,5561 |
| 116 | $C_4H_9$-tert | $CH_3$ | 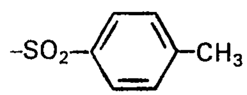 | 1 | 85 | $n_D^{22}$:1,5509 |
| 117 | $C_4H_9$-sec | $CH_3$ | 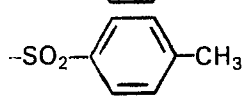 | 1 | 91 | $n_D^{22}$: 1,5508 |
| 117a | $C_3H_7$-n | $CH_3$ | 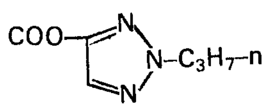 | 1 | 82 | $n_D^{18}$:1,5275 |
| 117b | $C_4H_9$-n | $CH_3$ | 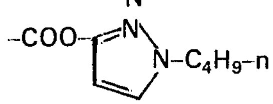 | 1 | 88 | $n_D^{18}$:1,5218 |
| 117b | $C_2H_5$ | $CH_3$ | 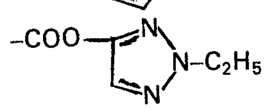 | 1 | 88 | 62 |

Beispiel 8

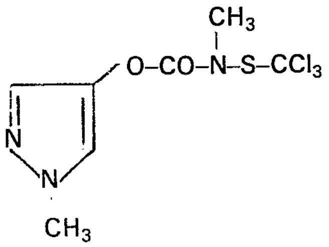

(162)

Zu einer Mischung aus 11,3 g (0,05 Mol) N-Methyl -N- trichlormethylthio-carbamoylfluorid, 4,9 g (0,05 Mol) 1-Methyl-4-hydroxypyrazol und 150 ml Toluol tropft man 5,1 g (0,05 Mol) Triethylamin. Nach 4 Stunden wird zweimal mit je 25 ml Wasser extrahiert. Die organische Phase wird über Natriumsulfat getrocknet und im Vakuum eingedampft. Den Rückstand destilliert man im Hochvakuum an. Man erhält so 12,5 g (82% der Theorie) n-Methyl-N-trichlormethylthio-O-[1-methyl-pyrazol(4)yl] -carbaminsäureester in Form eines gelben Öles mit dem Brechungsindex $n_D^{23}$:1,5492.

Analog Beispiel 8 können die folgenden Verbindungen der Formel

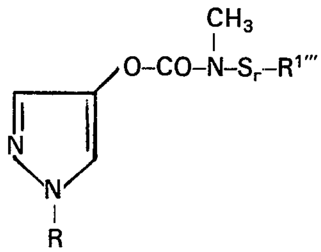

(Ic)

hergestellt werden:

| Produkt Nr. | R | R1''' | r | Ausbeute (% der Theorie) | Brechungsindex; Schmelzpunkt °C |
|---|---|---|---|---|---|
| 118 | $C_3H_7$-iso | $CCl_2F$ | 1 | 89 | $n_D^{18}$:1,5122 |
| 119 | $C_4H_9$-tert. | $CCl_3$ | 1 | 92 | 56 |
| 120 | $C_4H_9$-tert. | $CCl_2F$ | 1 | 85 | $n_D^{18}$: 1,5108 |
| 121 | $C_3H_7$-iso | $CH_3$ | 1 | | |
| 122 | $C_3H_7$-iso | 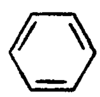 | 1 | | |
| 123 | $C_3H_7$-iso | $CH_2$-$CH=CH_2$ | 1 | | |
| 124 | $C_4H_9$-iso | $CCl_3$ | 1 | | |
| 125 | $C_4H_9$-iso | $CCl_2F$ | 1 | 91 | $n_D^{28}$:1,5051 |
| 126 | $C_4H_9$-sek. | $CCl_3$ | 1 | 92 | $n_D^{25}$: 1,5283 |
| 127 | $C_4H_9$-sek. | $CCl_2F$ | 1 | 61 | $n_D^{19}$:1,50 92 |
| 128 | $C_3H_7$-iso | $CCl_3$ | 1 | 79 | $n_D^{21}$:1,5378 |
| 129 | $C_2H_5$ | $CCl_3$ | 1 | 94 | $n_D^{26}$:1,5392 |
| 130 | $C_4H_9$-sek. | $CH_2$–$C=CH$ | 1 | | |
| 131 | $C_3H_7$-n | $CCl_2F$ | 1 | 95 | $n_D^{28}$:1,5082 |
| 132 | $C_4H_9$-n | $CCl_3$ | 1 | | |
| 133 | $C_3H_7$-iso | $C_3H_7$-n | 1 | | |
| 134 | $CH_2=CH$–$CH_2$ | $CCl_3$ | 1 | | |
| 135 | $CH=C$–$CH_2$ | $CCl_3$ | 1 | | |
| 136 | 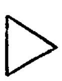 | $CCl_2F$ | 1 | | |
| 137 | 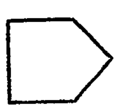 | $CCl_2F$ | 1 | 95 | $n_D^{28}$:1,5259 |

(Fortsetzung)

| Produkt Nr. | R | $R^{1'''}$ | r | Ausbeute (% der Theorie) | Brechungsindex; Schmelzpunkt °C |
|---|---|---|---|---|---|
| 138 | $C_2H_5O-CH_2-CH_2$ | $CCl_2F$ | 1 | | |
| 139 | $NC-CH_2-CH_2$ | $CCl_2F$ | 1 | | |
| 140 | $NC-CH_2-CH_2$ | $CCl_3$ | 1 | | |
| 141 | $C_2H_5O-CH_2-CH_2$ | $CCl_3$ | 1 | | |
| 142 | $C_4H_9$-tert. | $CH_3$ | 1 | | |
| 143 | $C_3H_7$-iso | $CH_3$ | 2 | | |
| 144 | $C_3H_7$-iso | $CH_2$— 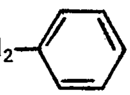 | 1 | | |
| 145 | $CH(C_2H_5)_2$ | $CCl_2F$ | 1 | | |
| 146 | $CH_3-S-CH_2$ | $CCl_2F$ | 1 | | |
| 147 | $CH_3-S-CH_2$ | $CCl_3$ | 1 | | |
| 148 | $C_4H_9$-tert. | $CH_2-CH=CH_2$ | 1 | | |
| 149 | $C_4H_9$-n | $CCl_2F$ | 1 | 97 | $n_D^{28}$D:1,5052 |
| 150 | $C_2H_5$ | $CCl_2F$ | 1 | 93 | $n_D^{28}$:1,5130 |
| 151 | $CH_2-C(CH_3)_3$ | $CCl_2F$ | 1 | | |
| 152 | $C(CH_3)_2-CH_2-CH_3$ | $CCl_2F$ | 1 | 94 | $n_D^{23}$:1,5071 |
| 153 | $CH_2=CH-CH_2$ | $CCl_2F$ | 1 | | |
| 154 | $CH=C-CH_2$ | $CCl_2F$ | 1 | | |
| 155 | $C_3H_7$-iso | $CClF_2$ | 1 | | |
| 156 | $C_4H_9$-tert. | $CClF_2$ | 1 | | |
| 157 | $C(CH_3)_2-CH_2-CH_3$ | $CCl_3$ | 1 | | |
| 158 | $C_3H_7$-iso | $C_3H_7$-n | 1 | | |
| 159 | $CH_3$ | $CCl_2F$ | 1 | 87 | $n_D^{28}$:1,5196 |
| 160 | 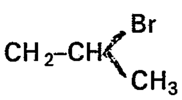 | $CCl_2F$ | 1 | 82 | $n_D^{19}$:1,5352 |
| 161 | $CH_2-CH_2-Br$ | $CCl_2F$ | 1 | 88 | $n_D^{22}$:1,5416 |

Beispiel A

Phaedon-Larven-Test

Lösungsmittel: 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, dass alle Käfer-Larven abgetötet wurden; 0% bedeutet, dass keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 4, 6, 7, 11, 14, 17, 18, 20, 22, 25, 29, 31, 33, 36, 39, 40, 50, 51, 57, 63, 67, 93, 127, 103, 128, 118, 119, 120, 106, 70, 66, 92, 77, 90, 88, 79, 75, 83, 85, 61, 107, 81, 101.

Beispiel B

Grenzkonzentrations-Test / Bodeninsekten

Testinsekt: Phorbia antiqua-Maden (im Boden)

Lösungsmittel: 3 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den Boden in Töpfe und lässt diese bei Raumtemperatur stehen.

Nach 24 Stunden werden die Testtiere in den behandelten Boden gegeben und nach weiteren 2 bis 7 Tagen wird der Wirkungsgrad des Wirkstoffes durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 67, 50, 51, 36, 40, 57, 128.

Beispiel C
Grenzkonzentrations-Test/Wurzelsystemische Wirkung

| | |
|---|---|
| Testinsekt: | Myzus persicae |
| Lösungsmittel: | 3 Gewichtsteile Aceton |
| Emulgator: | 1 Gewichtsteil Alkylarylpolyglykoläther |

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschliesslich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 67, 50, 51, 36, 40, 57, 128.

Beispiel D
Grenzkonzentrations-Test/Nematoden

| | |
|---|---|
| Testnematode: | Meloidogyne incognita |
| Lösungsmittel: | 3 Gewichtsteile Aceton |
| Emulgator: | 1 Gewichtsteil Alkylarylpolyglykoläther |

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27 °C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100%, wenn der Befall vollständig vermieden wird, er ist 0%, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik: 67.

Beispiel E
$LT_{100}$-Test für Dipteren

| | |
|---|---|
| Testtiere: | Aedes aegypti |
| Zahl der Testtiere: | 25 |
| Lösungsmittel: | Aceton |

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschliessend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Bei diesem Test zeigen z.B. die folgenden Verbindungen der Herstellungsbeispiele überlegene Wirkung gegenüber dem Stand der Technik: 14, 33, 31, 25, 17, 128, 11, 20, 39, 51, 29, 36, 10, 7, 18, 40, 57, 67, 50.

**Patentansprüche**

1. N-acylierte N-Methyl-O-pyrazol(4)yl- carbaminsäureester der Formel

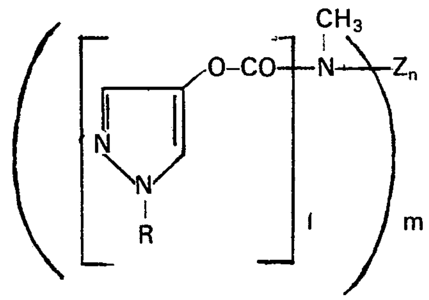

(I)

in welcher

R für gegebenenfalls durch Halogen, Cyano, $C_1$–$C_4$-Alkoxy oder $C_1$–$C_4$-Alkylthio substituiertes $C_1$–$C_5$-Alkyl für $C_3$–$C_5$-Alkenyl, $C_3$–$C_5$-Alkinyl, $C_3$–$C_6$-Cycloalkyl oder für gegebenenfalls halogen-substituiertes Phenyl, Benzyl oder Phenylethyl steht,

Z für die Reste –S–, –S–S–, –CO–$R^1$ oder –$S_r(O)_sR^4$ steht, wobei $R^1$ r, s, $R^4$ die unten angegebe Bedeutung haben und

l für 1 oder 2,

m für 1 oder 2,

n für 0 oder 1 steht, wobei für den Fall, dass l, m und n jeweils für 1 stehen,

Z für den Rest –CO–$R^1$ steht, worin

$R^1$ für Halogen, $C_1$–$C_4$-Alkoxy, $C_3$–$C_5$-Alkenoxy, $C_3$–$C_5$ -Alkinoxy, $C_1$–$C_4$-Alkylthio, $C_1$–$C_4$-Monoalkylamino, $C_1$–$C_4$-Dialkyl-amino, $C_1$–$C_4$-Alkyl-hydroxylamino, für gegebenenfalls durch Halogen, Nitro, Cyano, Trifluormethyl, $C_1$–$C_4$-Alkyl, $C_1$–$C_4$-Alkoxy, $C_1$–$C_4$-Alkylendioxyl, $C_1$–$C_4$-Alkylthio, $C_1$–$C_4$-Alkoxy-carbonyl-substituiertes Phenoxy, Phenylthio oder Phenylamino, für 2,3-Dihydro-2,2-dimethyl-7-benzofuranyl oder für den Rest

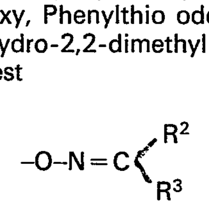

steht, worin

$R^2$ für Wasserstoff, $C_1$–$C_4$-Alkyl oder $C_1$–$C_4$-Dialkylamino-carbonyl steht und

$R^3$ für $C_1$–$C_4$-Alkyl, $C_1$–$C_4$-Alkylthio, Cyano-$C_1$–$C_4$-alkylthio, $C_1$–$C_4$-Alkylthio-$C_1$–$C_4$-alkyl steht oder die beiden Reste $R^2$ und $R^3$ zusammen für gegebenenfalls durch Sauerstoff, Schwefel, SO oder $SO_2$ unterbrochenes $C_2$–$C_8$-Alkandiyl stehen, wobei für den Fall, dass l, m und n jeweils für 1 stehen,

Z für den Rest –$S_r(O)_sR^4$ steht, worin r für 1 oder 2 und s für Null, 1 oder 2 stehen und

$R^4$ für gegebenenfalls durch Halogen substituiertes $C_1$–$C_4$-Alkyl, $C_3$–$C_5$-Alkenyl, $C_3$–$C_5$-Alkinyl oder $C_3$–$C_6$-Cycloalkyl, für gegebenenfalls durch Halogen, Cyano, Nitro, Trifluormethyl, $C_1$–$C_4$-Alkyl oder $C_1$–$C_4$-Alkoxy substituiertes Phenyl, Benzyl oder Phenylethyl oder für den Rest

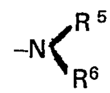

steht, worin

$R^5$ für $C_1$–$C_4$-Alkyl, $C_3$–$C_5$-Alkenyl, $C_3$–$C_5$-Alkinyl, $C_3$–$C_6$-Cycloalkyl oder Benzyl steht und

$R^6$ für $C_1$–$C_4$-Alkyl, $C_3$–$C_5$-Alkenyl, $C_3$–$C_5$-Alkinyl, $C_3$–$C_6$-Cycloalkyl, Benzyl, Phenylethyl, Halogencarbonyl, Formyl, $C_1$–$C_4$-Alkyl-carbonyl, $C_1$-$C_4$-alkoxy-carbonyl, $C_1$–$C_4$-Alkoxy-phenoxy-carbonyl, $C_3$–$C_5$-Alkinoxy-carbonyl, $C_3$–$C_5$-Alkenoxycarbonyl, $C_1$–$C_4$-Alkylthiocarbonyl, $C_1$–$C_4$-Alkyl-amino-carbonyl, $C_1$–$C_4$-Akyl -hydroxylaminocarbonyl, $C_1$–$C_{10}$-Alkyl-phenoxycarbonyl, Di-$C_1$-$C_4$-alkyl-amino-carbonyl, Phenylthiocarbonyl, Phenoxycarbonyl, 2,3-Dihydro-2,2-dimethyl-7-benzofuranyloxycarbonyl, für gegebenenfalls durch Halogen, Cyano, Nitro, Trifluormethyl, $C_1$–$C_{10}$-Alkyl oder $C_1$–$C_4$-Alkoxy substituiertes Phenylsulfenyl, Phenylsulfinyl, Phenylsulfonyl oder Phenyl steht oder für den Rest

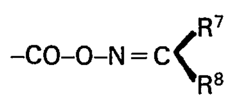

steht, worin

$R^7$ die oben für $R^2$ angegebene und

$R^8$ die oben für $R^3$ angegebene Bedeutung hat, wobei ferner im Rest 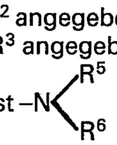 die Reste $R^5$ und $R^6$ zusammen für eine gegebenenfalls durch Sauerstoff oder Schwefel unterbrochene Kohlenwasserstoffkette mit 3 bis 8 Kohlenstoffatomen stehen.

2. Verbindungen gemäss Anspruch 1 wobei in Formel (I) R die in Anspruch 1 angegebene Bedeutung hat und entweder l für 2, m für 1 und n für Null steht, oder, für den Fall, dass Z für –S– oder –S–S– steht, l für 1, m für 2 und n für 1 steht.

3. Verbindung der Formel

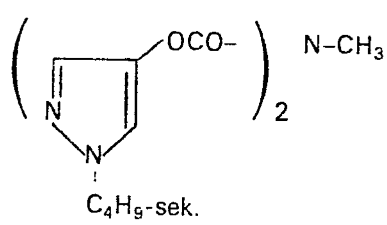

4. Verbindung der Formel

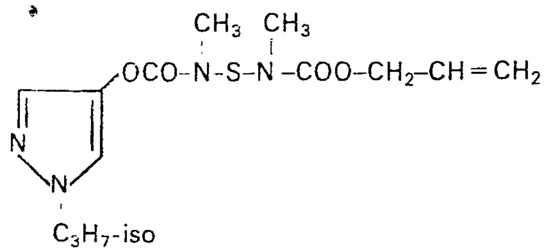

5. Verbindung der Formel

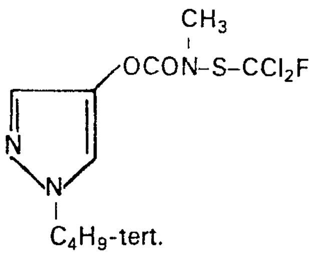

6. Verfahren zur Herstellung der Verbindungen der Formel (I) gemäss Anspruch 1,

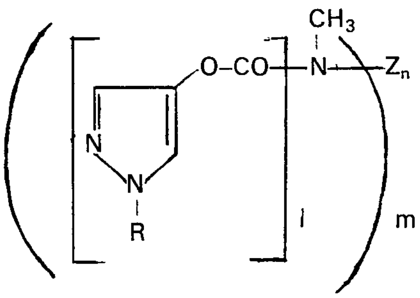

(I)

in welcher

R für gegebenenfalls durch Halogen, Cyano, $C_1$–$C_4$-Alkoxy oder $C_1$–$C_4$-Alkylthio substituiertes $C_1$–$C_5$-Alkyl für $C_3$–$C_5$-Alkenyl, $C_3$–$C_5$-Alkinyl, $C_3C_6$–Cycloalkyl oder für gegebenenfalls halogensubstituiertes Phenyl, Benzyl oder Phenylethyl steht,

Z für die Reste –S–, –S–S, –CO–$R^1$ oder $-S_r(O)_sR^4$ steht, wobei $R^1$, r, s, $R^4$ die unten angegebene Bedeutung haben und

l für 1 oder 2,

m für 1 oder 2,

n für 0 oder 1 steht, wobei für den Fall, dass l, m und n jeweils für 1 stehen,

Z für den Rest –CO–$R^1$ steht, worin

$R^1$ für Halogen, $C_1$–$C_4$-Alkoxy, $C_3$–$C_5$-Alkenoxy, $C_3$–$C_5$-Alkinoxy, $C_1$–$C_4$-Alkylthio, $C_1$–$C_4$-Monoalkylamino, $C_1$–$C_4$-Dialkyl-amino, $C_1$–$C_4$-Alkyl-hydroxylamino, für gegebenenfalls durch Halogen, Nitro, Cyano, Trifluormethyl, $C_1$–$C_4$-Alkyl, $C_1$–$C_4$-Alkoxy, $C_1$-$C_4$-Alkylendioxy, $C_1$–$C_4$-Alkylthio, $C_1$–$C_4$-Alkoxy-carbonyl-substituiertes Phenoxy, Phenylthio oder Phenylamino, für 2,3-Dihydro-2,2-dimethyl-7-benzofuranyl oder für den Rest

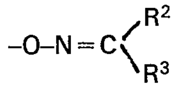

steht, worin

$R^2$ für Wasserstoff, $C_1$–$C_4$-Alkyl oder $C_1$–$C_4$-Dialkylamino-carbonyl steht und

$R^3$ für $C_1$–$C_4$-Alkyl, $C_1$–$C_4$-Alkylthio, Cyano-$C_1$–$C_4$-alkylthio, $C_1$–$C_4$-Alkylthio–$C_1$–$C_4$ -alkyl steht oder die beiden Reste $R^2$ und $R^3$ zusammen für gegebenenfalls durch Sauerstoff, Schwefel, SO oder $SO_2$ unterbrochenes $C_2$–$C_8$-Alkandiyl stehen, wobei für den Fall, dass 1, m und n jeweils für 1 stehen,

Z für den Rest $-S_r(O)_sR^4$ steht, worin r für 1 oder 2 und s für Null, 1 oder 2 stehen und

$R^4$ für gegebenenfalls durch Halogen substituiertes $C_1$–$C_4$-Alkyl, $C_3$–$C_5$-Alkenyl, $C_3$–$C_5$-Alkinyl oder $C_3$–$C_6$-Cycloalkyl, für gegebenenfalls durch Halogen, Cyano, Nitro, Trifluormethyl, $C_1$–$C_4$-Alkyl oder $C_1$–$C_4$-Alkoxy substituiertes Phenyl, Benzyl oder Phenylethyl oder für den Rest

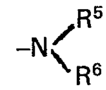

steht, worin

$R^5$ für $C_1$–$C_4$-Alkyl, $C_3$–$C_5$-Alkenyl, $C_3$–$C_5$-Alkinyl, $C_3$–$C_6$-Cycloalkyl oder Benzyl steht und

$R^6$ für $C_1$–$C_4$-Alkyl, $C_3$–$C_5$-Alkenyl, $C_3$-Alkinyl, $C_3$–$C_6$-Cycloalkyl, Benzyl, Phenylethyl, Halogencarbonyl, Formyl, $C_1$–$C_4$-Alkyl-carbonyl, $C_1$–$C_4$-alkoxy-carbonyl, $C_1$–$C_4$-Alkoxy-phenoxy-carbonyl, $C_3$–$C_5$-Alkinoxy-carbonyl, $C_3$–$C_5$-Alkenoxy-carbonyl, $C_1$–$C_4$-Alkylthiocarbonyl, $C_1C_4$-Alkylamino-carbonyl, $C_1$–$C_4$-Alkyl-hydroxylaminocarbonyl, $C_1$–$C_{10}$-Alkyl-phenoxy-carbonyl, Di–$C_1$-$C_4$-alkylamino-carbonyl, Phenylthiocarbonyl, Phenoxycarbonyl, 2,3-Dihydro-2,2-dimethyl-7-benzofuranyl-oxycarbonyl, für gegebenenfalls durch Halogen, Cyano, Nitro, Trifluormethyl, $C_1$–$C_{10}$-Alkyl oder $C_1$–$C_4$-Alkoxy substituiertes Phenylsulfenyl, Phenylsulfinyl, Phenylsulfonyl oder Phenyl steht oder für den Rest

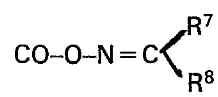

steht, worin

$R^7$ die oben für $R^2$ angegebene und

$R^8$ die oben für $R^3$ angegebene Bedeutung hat,

wobei ferner im Rest 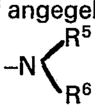 die Reste $R^5$ und $R^6$ zusammen für eine gegebenenfalls durch Sauerstoff oder Schwefel unterbrochene Kohlenwasserstoffkette mit 3 bis 8 Kohlenstoffatomen stehen, dadurch gekennzeichnet, dass

(a) man N-Methyl-O-pyrazol(4)yl-carbaminsäureester der Formel II

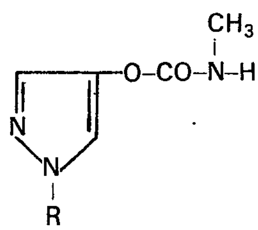

(II)

in welcher

R die oben angegebene Bedeutung hat, mit Acylierungsmitteln der Formel III

$$X{-}Z_n \qquad (III)$$

in welcher

Z die oben angegebene Bedeutung hat,

X für Fluor, Chlor oder Brom steht und

n für 1 steht oder, mit Schwefeldichlorid oder Dischwefeldichlorid, gegebenenfalls in Gegenwart eines Säureakzeptors und gegebenenfalls unter Verwendung eines Verdünnungsmittels umsetzt, oder

(b) man 4-Hydroxy-pyrazole der Formel IV

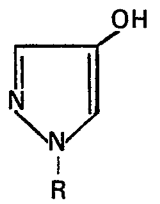

(IV)

in welcher

R die oben angegebene Bedeutung hat, mit Acylierungsmitteln der Formel V

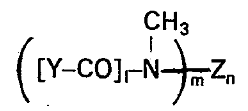

(V)

in welcher

Z, l, m und n die oben angegebene Bedeutung haben und

Y für Fluor oder Chlor steht, gegebenenfalls in Gegenwart eines Säureakzeptors und gegebenenfalls unter Verwendung eines Verdünnungsmittels und gegebenenfalls in Gegenwart einer weiteren carbamoylierbaren Verbindung umsetzt.

7. Schädlingsbekämpfungsmittel, gekennzeichnet durch einen Gehalt an mindestens einem N-acylierten N-Methyl- O-pyrazol(4)- yl-carbaminsäureester der Formel (I).

8. Verwendung von N-acylierten N-methyl-O-pyrazol(4)-yl carbaminsäureestern der Formel (I) zur Bekämpfung von Schädlingen.

9. Verfahren zur Herstellung von Schädlingsbekämpfungsmitteln, dadurch gekennzeichnet, dass man N-acylierte N-Methyl-O-pyrazol(4)yl-carbaminsäureester der Formel (I) mit Streckmitteln und/oder oberflächenaktiven Mitteln vermischt.

## Revendications

1. Esters de O-pyrazole(4)yle d'acides N-méthyl-carbamiques N-acylés de formule

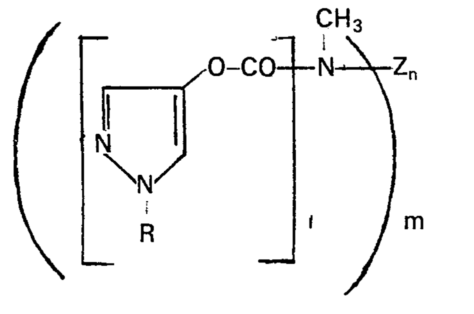

(I)

dans laquelle

R désigne un groupe alkyle en $C_1$ à $C_5$ éventuellement substitué par un radial halogéno, cyano, alkoxy en $C_1$ à $C_4$ ou alkylthio en $C_1$ à $C_4$, un groupe alcényle en $C_3$ à $C_5$, un groupe alcynyle en $C_3$ à $C_5$, un groupe cycloalkyle en $C_3$ à $C_6$ ou un groupe phényle, benzyle ou phényléthyle éventuellement halogéné,

Z représente les restes –S–, –S–S, $-CO-R^1$ ou $-S_r(O)_sR^4m$ où $R^1$, r, s, $R^4$ ont la définition indiquée ci-dessous et

l est égal à 1 ou à 2,

m est égal à 1 ou à 2,

n est égal à 0 ou à 1

au cas où chacun de l, m et n est égal à 1,

Z désigne le reste $-CO-R^1$, où

$R^1$ désigne de l'halogène, un groupe alkoxy en $C_1$ à $C_4$ alcénoxy en $C_3$ à $C_5$ alcynoxy en $C_3$ à $C_5$, alkylthio en $C_1$ à $C_4$, monoalkylamino en $C_1$ à $C_4$, di-(alkyle en $C_1$ à $C_4$)-amino, alkylhydroxylamino en $C_1$ à $C_4$, un groupe phénoxy, phénylthio ou phénylamino éventuellement substitué par un radical halogéno ou nitro, cyano, trifluorométhyle, alkyle en $C_1$ à $C_4$, alkoxy en $C_1$ à $C_4$, alkylènedioxy en $C_1$ à $C_4$, alkylthio en $C_1$ à $C_4$, (alkoxy en $C_1$ à $C_4$) carbonyle, un groupe 2,3-dihydro-2,2-diméthyl-7-benzofurannyle ou le reste

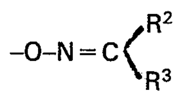

dans lequel

$R^2$ désigne l'hydrogène, un groupe alkyle en $C_1$ à $C_4$ ou un groupe di-(alkyle en $C_1$ à $C_4$)-aminocarbonyle et

$R^3$ est un groupe alkyle en $C_1$ à $C_4$, alkylthio en $C_1$ à $C_4$, cyano-(alkyle en $C_1$ à $C_4$)thio, (alkylthio en $C_1$ à $C_4$)-alkyle en $C_1$ à $C_4$, ou bien les deux restes $R^2$ et $R^3$ forment ensemble un groupe alcanediyle en $C_2$ à $C_8$ éventuellement interrompu par de l'oxygène, du souffre, un groupe SO ou un groupe $SO_2$, au cas où chacun de l, m et n est égal à 1,

Z représente le reste $-S_r(O)_sR^4$, dans lequel r est égal à 1 ou à 2 et s est égal à zéro, 1 ou 2 et

$R^4$ désigne un groupe alkyle en $C_1$ à $C_4$, alcényle en $C_3$ à $C_5$, alcynyle en $C_3$ à $C_5$ ou cycloalkyle en $C_3$ à $C_6$ éventuellement substitué par un halogène, un groupe phényle, benzyle ou phényléthyle éventuellement substitué par un radical halogéno, cyano, nitro, trifluorométhyle, alkyle en $C_1$ à $C_4$ ou alkoxy en $C_1$ à $C_4$ ou le reste de formule

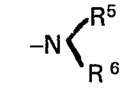

dans laquelle

$R^5$ est un groupe alkyle en $C_1$ à $C_4$, alcényle en $C_3$ à $C_5$, alcynyle en $C_3$ à $C_5$, cycloalkyle en $C_3$ à $C_6$ ou benzyle et

$R^6$ est un groupe alkyle en $C_1$ à $C_4$, alcényle en

$C_3$ à $C_5$, alcynyle en $C_3$ à $C_5$, cycloalkyle en $C_3$ à $C_6$, benzyle, phényléthyle, halogénocarbonyle, formyle, (alkyle en $C_1$ à $C_4$ )carbonyle, (alkoxy en $C_1$ à $C_4$)carbonyle, (alkoxy en $C_1$ à $C_4$)phénoxycarbonyle, (alcynoxy en $C_3$ à $C_5$)carbonyle, (alcénoxy en $C_3$ à $C_5$)carbonyle, (alkylthio en $C_1$ à $C_4$) carbonyle, (alkyle en $C_1$ à $C_4$)aminocarbonyle, (alkyle en $C_1$ à $C_4$)hydroxylaminocarbonyle, (alkyle en $C_1$ à $C_{10}$) phénoxycarbonyle, di(alkyle en $C_1$ à $C_4$)aminocarbonyle, phénylthiocarbonyle, phénoxy- carbonyle, 2,3-dihydro -2,2- di méthyl-7 -benzo- furannyloxycarbonyle, un groupe phénylsulfényle, phénylsulfinyle, phénylsulfonyle ou phényle éventuellement substitué par un radical halogéno, cyano, nitro, trifluorométhyle, alkyle en $C_1$ à $C_{10}$ ou alkoxy en $C_1$ à $C_4$, ou le reste de formule

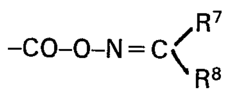

dans laquelle
$R^7$ a la définition indiquée ci-dessus pour $R^2$ et
$R^8$ a la définition indiquée ci-dessus pour $R^3$, en outre, dans le reste

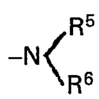

les restes $R^5$ et $R^6$ forment ensemble une chaîne hydrocarbonée éventuellement interrompue par de l'oxygène ou du soufre et ayant 3 à 8 atomes de carbone.

2. Composés suivant la revendication 1, dans la formule (I) desquels
R a la définition indiquée dans la revendication 1 et l est égal à 2, m est égal à 1 ou n est égal à zéro ou bien, au cas où Z représente –S–ou –S–S–, l est égal à 1, m est égal à 2 et n est égal à 1.

3. Composé de formule

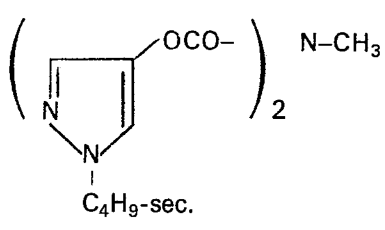

4. Composé de formule

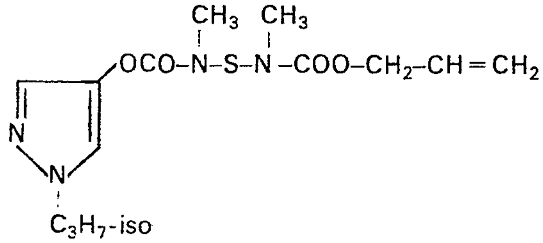

5. Composé de formule

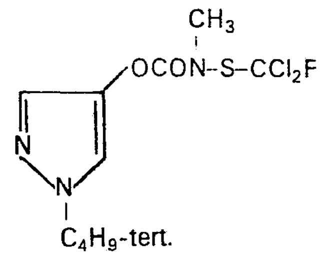

6. Procédé de production des composés de formule (I) suivant la revendication 1

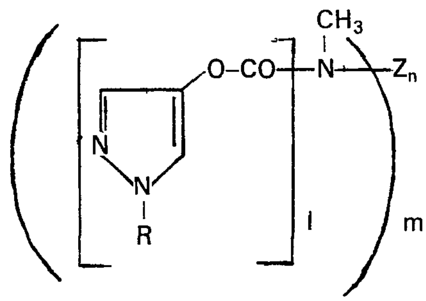

(I)

dans laquelle
R désigne un groupe alkyle en $C_1$ à $C_5$ éventuellement substitué par un radical halogéno, cyano, alkoxy en $C_1$ à $C_4$ ou alkylthio en $C_1$ à $C_4$, un groupe alcényle en $C_3$ à $C_5$, un groupe alcynyle en $C_3$ à $C_5$, un groupe cycloalkyle en $C_3$ à $C_6$ ou un groupe phényle, benzyle ou phényléthyle éventuellement halogéné,
Z représente les restes –S–, –S–S–, –CO–$R^1$ ou $-S_r(O)_sR^4$, où $R^1$, r, s, $R^4$ ont la définition indiquée ci-dessous et
l est égal à 1 ou à 2,
m est égal à 1 ou à 2,
n est égal à 0 ou á 1
au cas où chacun de l, m et n est égal à 1,
Z désigne le reste –CO–$R^1$, où
$R^1$ désigne un halogène, une groupe alkoxy en $C_1$ à $C_4$, alcénoxy en $C_3$ à $C_5$, alcynoxy en $C_3$ à $C_5$, alkylthio en $C_1$ à $C_4$, monoalkylamino en $C_1$ à $C_4$, di-(alkyle en $C_1$ à $C_4$)-amino, alkylhydroxylamino en $C_1$ à $C_4$, un groupe phénoxy, phénylthio ou phénylamino éventuellement substitué par un radical halogéno ou nitro, cyano, trifluorométhyle, alkyle en $C_1$ à $C_4$, alkoxy en $C_1$ à $C_4$, alkylènedioxy en $C_1$ à $C_4$, alkylthio en $C_1$ à $C_4$, (alkoxy en $C_1$ à $C_4$)carbonyle, un groupe 2,3-dihydro-2,2-diméthyl-7-benzofurannyle ou le reste

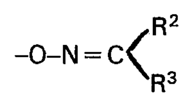

dans laquel
$R^2$ désigne l'hydrogène, un groupe alkyle en $C_1$ à $C_4$ ou un groupe di-(alkyle en $C_1$ à $C_4$)-aminocarbonyle et
$R^3$ est un groupe alkyle en $C_1$ à $C_4$, alkylthio en $C_1$ à $C_4$, cyano-(alkyle en $C_1$ à $C_4$)thio, (alkylthio en $C_1$ à $C_4$)-alkyle en $C_1$ à $C_4$,ou bien les deux res-

tes $R^2$ et $R^3$ forment ensemble un groupe alcanediyle en $C_2$ à $C_8$ éventuellement interrompu par de l'oyygène, du souffre, un groupe SO un groupe $SO_2$, au cas où chacun de l, m et n est égal à 1,

Z représente le reste $-S_r(O)_sR^4$, dans lequel r est égal à 1 ou à 2 et s est égal à zéro, 1 ou 2 et

$R^4$ désigne un groupe alkyle en $C_1$ à $C_4$, alcényle en $C_3$ à $C_5$, alcynyle en $C_3$ à $C_5$ ou cycloalkyle en $C_3$ à $C_6$ éventuellement substitué par un halogène, un groupe phényle, benzyle ou phényléthyle éventuellement substitué par un radical halogéno, cyano, nitro, trifluorométhyle, alkyle en $C_1$ à $C_4$ ou alkoxy en $C_1$ à $C_4$ ou le reste de formule

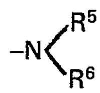

dans laquelle

$R^5$ est un groupe alkyle en $C_1$ à $C_4$, alcényle en $C_3$ à $C_5$, alcynyle en $C_3$ à $C_5$, cycloalkyle en $C_3$ à $C_6$ ou benzyle et

$R^6$ est une groupe alkyle en $C_1$ à $C_4$, alcényle en $C_3$ à $C_5$, alcynyle en $C_3$ à $C_5$m, cycloalkyle en $C_3$ à $C_6$, benzyle, phényléthyle, halogénocarbonyle, formyle, (alkyle en $C_1$ à $C_4$) carbonyle, (alkoxy en $C_1$ à $C_4$) carbonyle, (alkoxy en $C_1$ à $C_4$) phénoxycarbonyle, (alcynoxy en $C_3$ à $C_5$) carbonyle, (alcénoxy en $C_3$ à $C_5$) carbonyle, (alkylthio en $C_1$ à $C_4$) carbonyle, (alkyle en $C_1$ à $C_4$) aminocarbonyle, (alkyle en $C_1$ à $C_4$) hydroxylaminocarbonyle, (alkyle en $C_1$ à $C_{10}$) phénoxycarbonyle, di(alkyle en $C_1$ à $C_4$) amino carbonyle, phénylthiocarbonyle, phénoxycarbonyle, 2,3-dihydro-2,2-diméthyl -7- benzofurannyloxycarbonyle, un groupe phénylsulfényle, phénylsulfinyle, phénylsulfonyle ou phényle éventuellement substitué par un radical halogéno, cyano, nitro, trifluorométhyle, alkyle en $C_1$ à $C_{10}$ ou alkoxy en $C_1$ à $C_4$, ou le reste de formule

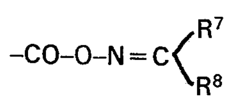

dans laquelle

$R^7$ a la définition indiquée ci-dessus pour $R^2$ et

$R^8$ a la définition indiquée ci-dessus pour $R^3$, en outre, dans le reste

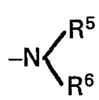

les restes $R^5$ et $R^6$ forment ensemble une chaîne hydrocarbonée éventuellement interrompue par de l'oxygène ou du soufre et ayant 3 à atomes de carbone, caractérisé en ce que

(a) on fait réagir un ester de O-pyrazole(4)yle d'acide N-méthylcarbamique de formule II

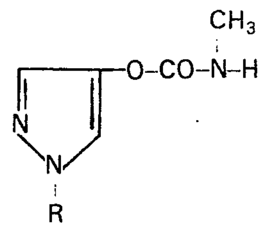

(II)

dans laquelle

R a la définition indiquée ci-dessus, avec des agents acylants de formule III

$X{-}Z_n$ (III)

dans laquelle

Z a la définition indiqué ci-dessus,

X représente du fluor, du chlore ou du brome et

n est égal à 1, ou avec du bichlorure de soufre ou du chlorure de soufre éventuellement en présence d'un accepteur d'acide et, le cas échéant, en utilisant un diluent, ou bien

(b) on fait réagir des 4-hydroxypyrazoles de formule IV

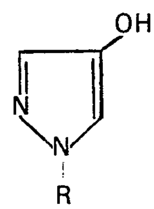

(IV)

dans laquelle

R a la définition indiquée ci-dessus, avec des agents acylants de formule V

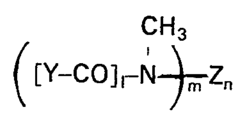

(V)

dans laquelle

Z, l, m et n ont la définition indiquée ci-dessus et

représente du fluor ou du chlore, éventuellement en présence d'un accepteur d'acide et, le cas échéant, en utilisant un diluant et en la présence éventuelle d'un autre composé être carbamoylé.

7. Composition pesticides, caractérisés par une teneur en au moins un ester de O-pyrazole(4)yle d'acide N-méthylcarbamique N-acilé de formule (I).

8. Utilisation des esters de O-pyrazole(4)yle d'acides N-méthylcarbamiques N-acylés de formule (I) pour combattre des parasites.

9. Procédé de préparation de compositions pesticides, caractérisé en ce qu'on mélange des esters de O-pyrazole(4)yle d'acides N-méthylcarbamiques N-acylés de formule (I) avec des diluants et/ou avec des agents tensio-actifs.

## Claims

1. N-acylated N-methyl-carbamic acid O-pyrazol-4-yl esters of the formula

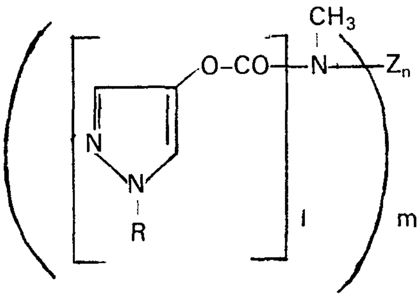

(I)

in which

R represents $C_1$–$C_5$-alkyl which is optionally substituted by halogen, cyano, $C_1$–$C_4$-alkoxy or $C_1$–$C_4$-alkylthio, or $C_3$–$C_5$-alkenyl, alkinyl or $C_3$–$C_6$-cycloalkyl, or optionally halogen-substituted phenyl, benzyl or phenylethyl,

Z represents the radicals –S–, –S–S–, –CO–$R^1$ or –$S_r(O)_sR^4$, wherein $R^1$, r, s and $R^4$ have the meaning given below, and

l represents 1 or 2,

m represents 1 or 2, and

n represents 0 or 1, wherein in the case where l, m and n each represents 1,

Z represents the radical –CO–$R^1$, wherein

$R^1$ represents halogen, $C_1$-$C_4$-alkoxy, $C_3$–$C_5$-alkenoxy, $C_3$–$C_5$-alkinoxy, $C_1$-$C_4$-alkylthio, $C_1$–$C_4$-monoalkylamino, $C_1$–$C_4$-dialkyl-amino or $C_1$–$C_4$-alkyl-hydroxylamino, or phenoxy, phenylthio or phenylamino which are optionally substituted by halogen, nitro, cyano, trifluoromethyl, $C_1$–$C_4$-alkyl, $C_1$–$C_4$-alkoxy, $C_1$–$C_4$-alkylenedioxy, $C_1$–$C_4$-alkylthio or $C_1$–$C_4$-alkoxy-carbonyl, or 2,3-dihydro-2,2-dimethyl-7-benzofuranyl or the radical

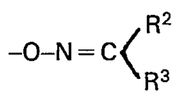

wherein

$R^2$ represents hydrogen, $C_1$–$C_4$-alkyl or dialkylaminocarbonyl and

$R^3$ represents $C_1$–$C_4$-alkyl, $C_1$–$C_4$-alkylthio, cyano–$C_1$–$C_4$-alkylthio or $C_1$–$C_4$-alkylthio–$C_1$–$C_4$-alkyl, or the two radicals $R^2$ and $R^3$ together represent $C_2$–$C_8$-alkanediyl which is optionally interrupted by oxygen, sulphur, SO, or $SO_2$, wherein in the case where l, m and n each represent 1,

Z represents the radical –$S_r(O)_sR^4$, wherein

r represents 1 or 2 and

s represents zero, 1 or 2 and

$R^4$ represents optionally halogen-substituted $C_1$–$C_4$-alkyl, alkenyl, $C_3$–$C_5$-alkinyl or $C_3$–$C_6$-cycloalkyl, or phenyl, benzyl or phenylethyl which are optionally substituted by halogen, cyano, nitro, trifluormethyl, $C_1$–$C_4$-alkyl or $C_1$–$C_4$alkoxy, or the radical

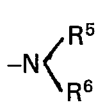

wherein

$R^5$ represents $C_1$–$C_4$-alkyl, $C_3$–$C_5$-alkenyl, $C_3$–$C_5$-alkinyl, $C_3$–$C_6$-cycloalkyl or benzyl and

$R^6$ represents $C_1$–$C_4$-alkyl, $C_3$–$C_5$-alkenyl, $C_3$–$C_5$-alkinyl, $C_3$–$C_6$-cycloalkyl, benzyl, phenylethyl, halogenocarbonyl, formyl, $C_1$–$C_4$-alkyl-carbonyl, $C_1$–$C_4$-alkoxy-carbonyl, $C_1$–$C_4$-alkoxy-phenoxy-carbonyl, $C_3$–$C_5$-alkinoxy-carbonyl, $C_3$–$C_5$-alkenoxy-carbonyl, $C_1$–$C_4$-alkylthiocarbonyl, $C_1$–$C_4$-alkyl-aminocarbonyl, $C_1$–$C_4$-alkyl-hydroxylaminocarbonyl, $C_1$–$C_{10}$-alkyl-phenoxy-carbonyl, di-$C_1$–$C_4$-alkylamino-carbonyl, phenylthiocarbonyl, phenoxycarbonyl or 2,3-dihydro-2,2-dimethyl-7-benzofuranyloxycarbonyl, or phenylsulphenyl, phenylsulphinyl, phenylsulphonyl or phenyl which are optionally substituted by halogen, cyano, nitro, trifluoromethyl, $C_1$–$C_{10}$-alkyl or $C_1$–$C_4$-alkoxy, or represents the radical

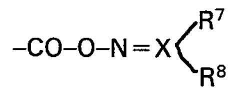

wherein

$R^7$ has the meaning given above for $R^2$ and

$R^8$ has the meaning given above for $R^3$, wherein, furthermore, in the radical

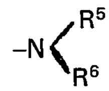

the radicals $R^5$ and $R^6$ together represent a hydrocarbon chain which has 3 to 8 carbon atoms and is optionally interrupted by oxygen or sulphur.

2. Compounds according to Claim 1, wherein in formula (I) R has the meaning given in Claim 1 and either l represents 2, m represents 1 and n represents zero, or in the case where Z represents –S– or –S–S–, 1 represents 1, m represents 2 and n represents 1.

3. Compound of the formula

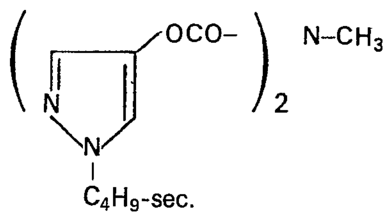

4. Compound of the formula

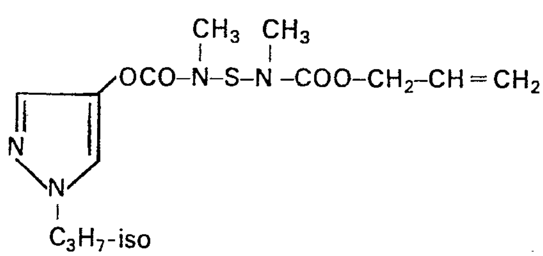

5. Compound of the formula

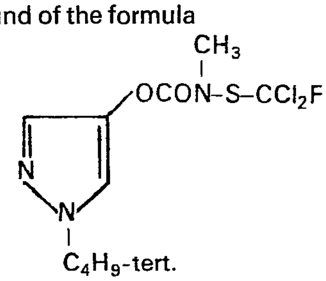

6. Process for the preparation of the compounds of the formula (I) according to claim 1,

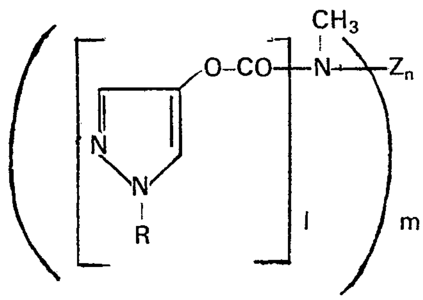

(I)

in which

R represents $C_1$–$C_5$-alkyl which is optionally substituted by halogen, cyano, $C_1$–$C_4$-alkoxy or $C_1$–$C_4$-alkylthio, or $C_3$–$C_5$-alkenyl, $C_3$–$C_5$-alkinyl or $C_3$–$C_6$-cycloalkyl, or optionally halogen-substituted phenyl, benzyl or phenylethyl

Z represents the radicals –S–, –S–S–, –CO–$R^1$ or –$S_r(0)_sR^4$, wherein $R^1$, r, s and $R^4$ have the meaning given below, and

l represents 1 or 2,

m represents 1 or 2, and

n represents 0 or 1, wherein in the case where l, m and n each represent 1,

Z represents the radical –CO–$R^1$, wherein

$R^1$ represents halogen, $C_1$–$C_4$-alkoxy, $C_3$–$C_5$-alkenoxy, $C_3$–$C_5$-alkinoxy, $C_1$–$C_4$-alkylthio, $C_1$–$C_4$-monoalkylamino, $C_1$–$C_4$-dialkyl-amino or $C_1$–$C_4$-alkyl-hydroxylamino, or phenoxy, phenylthio or phenylamino which are optionally substituted by halogen, nitro, cyano, trifluoromethyl, $C_1$–$C_4$-alkyl, $C_1$–$C_4$-alkoxy, $C_1$–$C_4$-alkylenedioxy, $C_1$–$C_4$-alkylthio or $C_1$–$C_4$-alkoxy-carbonyl, or 2,3-dihydro-2,2-dimethyl-7-benzofuranyl or the radical

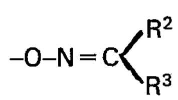

wherein

$R^2$ represents hydrogen, $C_1$–$C_4$-alkyl or $C_1$–$C_4$-dialkylaminocarbonyl and

$R^3$ represents $C_1$–$C_4$-alkyl, $C_1$–$C_4$-alkylthio, cyano-$C_1$–$C_4$-alkylthio or $C_1$–$C_4$ -alkylthio-$C_1$–$C_4$-alkyl, or the two radicals $R^2$ and $R^3$ together represent $C_2$–$C_8$-alkanediyl which is optionally interrupted by oxygen, sulphur, SO or $SO_2$, wherein in the case where l, m and n each represent 1,

Z represents the radical –$S_r(O)_sR^4$, wherein

r represents 1 or 2 and

s represents zero, 1 or 2 and

$R^4$ represents optionally halogen-substituted $C_1$–$C_4$-alkyl, $C_3$–$C_5$-alkenyl, $C_3$–$C_5$-alkinyl or $C_3$–$C_6$-cycloalkyl or phenyl, benzyl or phenylethyl which are optionally substituted by halogen, cyano, nitro, trifluoromethyl, $C_1$–$C_4$-alkyl or $C_1$–$C_4$-alkoxy, or the radical

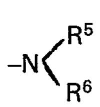

wherein

$R^5$ represents $C_1$–$C_4$-alkyl, $C_3$–$C_5$-alkenyl, $C_3$–$C_5$-alkinyl, $C_3$–$C_6$-cycloalkyl or benzyl and

$R^6$ represents $C_1$–$C_4$-alkyl, $C_3$–$C_5$-alkenyl, $C_3$–$C_5$-alkinyl, $C_3$–$C_6$-cycoalkyl, benzyl, phenylethyl, halogenocarbonyl, formyl, $C_1$–$C_4$-alkyl-carbonyl, $C_1$–$C_4$-alkoxy-carbonyl, $C_1$–$C_4$-alkoxy-phenoxy-carbonyl, $C_3$–$C_5$-alkinoxy-carbonyl, $C_3$–$C_5$-alkenoxy-carbonyl, $C_1$–$C_4$-alkylthiocarbonyl, $C_1$–$C_4$-alkyl-aminocarbonyl, $C_1$–$C_4$-alkyl-hydroxylaminocarbonyl, $C_1$–$C_{10}$-alkyl-phenoxy-carbonyl, di-$C_1$–$C_4$-alkylamino-carbonyl, phenylthiocarbonyl, phenoxycarbonyl or 2,3-dihydro-2,2-dimethyl-7-benzofuranyloxycarbonyl, or phenylsulphenyl, phenylsulphinyl, phenylsulphonyl or phenyl which are optionally substituted by halogen, cyano, nitro, trifluoromethyl, $C_1$–$C_{10}$-alkyl or $C_1$–$C_4$-alkoxy, or represents the radical

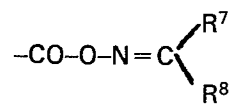

wherein

$R_7$ has the meaning given above for $R^2$ and

$R^8$ has the meaning given above for $R^3$, wherein, furthermore, in the radical

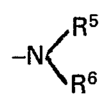

the radicales $R^5$ and $R^6$ together represent a hydrocarbon chain which has 3 to 8 carbon atoms and is optionally interrupted by oxygen or sulphur, characterised in that

(a) N-methyl-carbamic acid O-pyrazol-4-yl esters of the formula II

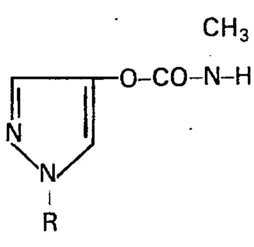

(II)

in which

R has the abovementioned meaning, are reacted with acylating agents of the formula III

$$X–Z_n \qquad (III)$$

in which

Z has the abovementioned meaning,

X represents fluorine, chlorine or bromine and

n represents 1, or with sulphur dichloride or disulphur chloride, if appropriate in the presence of an acid acceptor and if appropriate using a diluent, or

(b) 4-hydroxy-pyrazoles of the formula IV

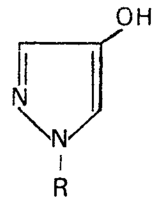

(IV)

in which

R has the abovementioned meaning, are reacted with acylating agents of the formula V

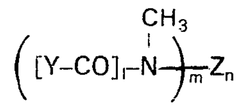

(V)

in which

Z, l, m and n have the abovementioned meaning and

Y represents fluorine or chlorine, if appropriate in the presence of an acid acceptor and if appropriate using a diluent and if appropriate in the presence of another compound which can be carbamoylated.

7. Agents for combating pests, characterised in that they contain at least one N-acylated N-methyl-carbamic acid O-pyrazol4-yl ester of the formula (I).

8. Use of N-acylated N-methyl-carbamic acid O-pyrazol-4-yl esters of the formula (I) for combating pests.

9. Process for the preparation of agents for combating pests, characterised in that N-acylated N-methyl-carbamic acid O-pyrazol-4-yl esters of the formula (I) are mixed with extenders and/or surface-active agents.